(19) Europäisches Patentamt
European Patent Office
Office européen des brevets

(11) **EP 1 842 554 A1**

(12) **EUROPEAN PATENT APPLICATION**
published in accordance with Art. 158(3) EPC

(43) Date of publication:
**10.10.2007 Bulletin 2007/41**

(51) Int Cl.:
*A61K 41/00* (2006.01)  *A61K 9/127* (2006.01)
*A61K 49/00* (2006.01)  *A61P 35/00* (2006.01)
*A61B 5/055* (2006.01)  *A61B 8/08* (2006.01)
*A61J 3/02* (2006.01)  *A61N 1/40* (2006.01)
*A61N 2/00* (2006.01)

(21) Application number: **06712038.6**

(22) Date of filing: **20.01.2006**

(86) International application number:
**PCT/JP2006/300813**

(87) International publication number:
**WO 2006/080243 (03.08.2006 Gazette 2006/31)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **28.01.2005 JP 2005021899**

(71) Applicant: **Konica Minolta Medical & Graphic, Inc. Tokyo 163-0512 (JP)**

(72) Inventor: **TAKEYAMA, Toshihisa machi, Hino-shi, Tokyo, 1918511 (JP)**

(74) Representative: **McCluskie, Gail Wilson et al J.A. Kemp & Co. 14 South Square Gray's Inn London WC1R 5JJ (GB)**

(54) **COATED MAGNETIC PARTICLE CONTAINING PREPARATION, PROCESS FOR PRODUCING THE SAME AND DIAGNOSTIC THERAPEUTIC SYSTEM**

(57)     It is an object of the invention to provide a pharmaceutical preparation containing magnetic vesicular particles which enables selective delivery of agents to a tumor site (targeting capability), is applicable to a contrast medium material or drug transport carrier and is also usable in thermotherapy employing heat-generation, and diagnosis and treatment of cancer by combinations thereof, and further a manufacturing method thereof and a diagnostic therapeutic system by use thereof.

There is disclosed a pharmaceutical preparation comprising magnetic vesicular particles, wherein the magnetic vesicular particles each include one or more magnetic microparticles within a lipid membrane, the magnetic microparticle having particulate gold attached to the surface of the magnetic microparticles and further having an organic compound bound to the particulate gold; and the vesicular particles meet the following requirement:

$$0.02 \leq R/(r \times 100) \leq 0.30$$

wherein R is an average particle size of the magnetic vesicular particles and r is an average particle size of the magnetic microparticles included in the magnetic vesicular particles.

FIG. 2

**Description**

**FIELD OF THE INVENTION**

The present invention relates to pharmaceutical preparations containing magnetic vesicular particles in which magnetic microparticles are covered with a lipid membrane, a particulate gold is attached to the magnetic microparticles and an organic compound containing a linking group is chemically bonded with the particulate gold, a pharmaceutical preparation containing the same and a manufacturing method thereof, and a diagnostic therapeutic system by use thereof.

**BACKGROUND OF THE INVENTION**

Development of "magnetic nano-beads" has been promoted as one of promising medical materials for use in next-generation medical techniques. The magnetic nano-beads (hereinafter, also denoted as magnetic microparticles) are nano-level size microparticles of ferrite (solid solution of $Fe_3O_4$ and $y-Fe_2O_3$). Recently, there has been developed a technique of synthesizing them under mild conditions of 4 to 25 °C in the range of a neutral pH, as described in JP-A No. 2002-128523 (hereinafter, the term, JP-A refers to Japanese Patent Application Publication). Magnetic nano-beads for medical use have been proposed, in which drugs or physiologically active materials are allowed to be fixed onto (attached to or included in) the magnetic microparticles covered with dextran, lipid, liposome, polymer or the like (as described in Patent document Nos. 1 and 2). As application of such magnetic microparticles in the medical field are proposed applications to contrast medium material used for MRI diagnosis or a medicine-transporting carrier, and thermotherapy employing heat generation due to hysteresis loss of magnetic microparticles in the high frequency magnetic field; there was further proposed concurrent performance of diagnosis and therapy of cancer by the combination of both of the foregoing (Non-patent document 1).

Thermotherapy for cancer (Hyperthermia) has been proposed for several decades and is one of the cancer therapies studied. The principle of therapy employs a property of cancer cells being weaker to heat than normal cells, thereby giving therapy with artificially maintaining a high temperature environment. Thermotherapy for cancer is a non-invasive treatment as compared to surgical removal operation which is generally conducted for cancer therapy. Comparing to chemotherapy or radiotherapy which often results in adverse effects, it is selective and its adverse effect becomes lower. Thus, it is a treatment which makes preservation of organs feasible and enhances the patient's quality of life. It is therefore a treatment suitable for an early cancer, or aged persons or infants who are intolerable to operative invasion or adverse effects. Conventional thermotherapy has employed techniques such as induction heating or focused ultrasonic heating and a recent alternant magnetic field heating using a ferrite type MRI contrast medium, and each of these therapies has merits and demerits. Accordingly, there is studied the possibility of using the above-described ferrite type particles as a novel heat-generating element in the alternating magnetic field (Non-patent document 1).

Feasibility of putting employment of magnetic microparticles into practice in the medical field relies on capability of maintaining a physiologically active material, a treatment drug or the like within the beads or capability of its selective delivery to an intended site (targeting capability). In this regard, the magnetic vesicular particles which have been proposed so far, still leave room for improvement. In enclosure of magnetic microparticles within liposome vesicles, various problems relating preparation of the liposome, for example, residue of organic solvents and stability of a liposome structure retard its practical use.

There have been also made studies of magnetic microparticles with thinking about the field of medical treatment/ diagnosis, biotechnology field and ecological field. For instance, a simple method of allowing noble metal ions of a noble metal complex to be attached onto the surface of magnetic microparticles upon exposure to ultrasonic or ionizing radiation was proposed (Patent document 3).

Patent document 1: WO 1998/040049
Patent document 2: JP-A No. 09-110722
Patent document 3: WO 2004/083124
Non-patent document 4: "BIO INDUSTRY" vol. 21, No. 8, page 48-54 (2004)

**SUMMARY OF THE INVENTION**

**PROBLEM TO BE SOLVED BY THE INVENTION**

It is an object of the present invention to provide a pharmaceutical preparation containing magnetic vesicular particles which enables selective delivery of agents to a tumor site (targeting capability), is applicable to a contrast medium material or drug transport carrier and is also usable in thermotherapy employing heat-generation, and diagnosis and treatment of cancer by combinations thereof, and further a manufacturing method thereof and a diagnostic therapeutic system by use thereof.

## MEANS FOR SOLVING THE PROBLEMS

One aspect of the invention is directed to a pharmaceutical preparation comprising magnetic vesicular particles, wherein the magnetic vesicular particles each include one or more magnetic microparticles within a lipid membrane, particulate gold is attached to surface of the magnetic microparticles and an organic compound is attached to the particulate gold via a linking group; and the vesicular particles meet the following requirement:

$$0.02 \leq R/(r \times 100) \leq 0.30$$

wherein R represents the average particle size of the magnetic vesicular particles and r represents the average particle size of all the magnetic microparticles included in the magnetic vesicular particles.

The vesicular particles preferably meet the following requirement:

$$0.03 \leq R/(r \times 100) \leq 0.30$$

wherein R represents the average particle size of the magnetic vesicular particles and r represents the average particle size of all the magnetic microparticles included in the magnetic vesicular particles.

The linking group is preferably selected from the group consisting of a mercapto group, dithio group, sulfo group, thiocarboxy group, dithiocarboxy group, amino group and hydroxyl group.

It is preferable that the average particle size of the magnetic microparticles be within the range of 5 nm to 30 nm and the main component of the magnetic microparticles be a ferrite.

It is preferable that the magnetic vesicular particles which include one or more magnetic microparticles within a lipid membrane are liposome particles and the surface electric charge of the liposome is positive.

The preparation containing magnetic vesicular particles are used preferably as an imaging agent and/or a therapeutic agent for tumor.

In the preparation containing magnetic vesicular particles, a physiologically active material and/or an antitumor-active material are bonded directly or via a linkage substance to the surface of the lipid membrane. The imaging agent is used preferably as a contrast medium for use in an ultrasonic imaging diagnostics, a nuclear magnetic resonance imaging diagnostics or an X-ray imaging diagnostics.

When the preparation containing magnetic vesicular particles is used as an imaging agent (e.g., a contrast medium) in an ultrasonic imaging diagnosis apparatus, a nuclear magnetic resonance imaging diagnosis apparatus or a radiographic image diagnosis apparatus, performing scans within not less than 1 min. and not more than 48 hr. after the preparation containing magnetic vesicular particles is dosed into the vein of an examinee, can result in enhanced capability to detect tumorous tissue.

On the other hand, when the preparation containing magnetic vesicular particles is injected into the region near tumorous tissue of an examinee, enhanced capability of detecting tumorous tissue can be achieved by performing scan within not less than 0.5 min. and not more than 36 hr. after the start of injection, using an ultrasonic imaging diagnostic apparatus, a nuclear magnetic resonance imaging diagnostic apparatus or a radiographic imaging diagnostic apparatus.

In the preparation containing magnetic vesicular particles, preferably, at least one selected from a physiologically functional material, additively stabilizing material, medicinally active material, medicinally active chelating material, antitumor-active material, immunopotentiating material, cell fusion material, and gene transfer mediating material is bonded directly or via a linking material to the outermost layer of the lipid membrane.

The above-described therapeutic agent is preferably an agent used for thermotherapy and the thermotherapy is performed, while being subjected to exposure to energy. The exposure to energy enables raising the temperature of the tumorous tissue in the close vicinity of the magnetic vesicular particles.

The exposure to energy is preferably exposure to an alternating magnetic field or exposure to ultrasonic waves and of these, exposure to an alternating magnetic field at a frequency of 25 to 500 kHz is specifically preferred.

When the preparation containing magnetic vesicular particles is used as a therapeutic agent, exposing an examinee to an alternating magnetic field or ultrasonic waves within not less than 1 min. and not more than 48 hr. after the preparation containing magnetic vesicular particles is dosed into the vein of the examinee, the temperature of a tumorous tissue can be raised in the close vicinity of the magnetic vesicular particles.

On the other hand, when the preparation containing magnetic vesicular particles is injected to the region near a tumorous tissue of an examinee for use as a therapeutic agent, exposure of the examinee to an alternating magnetic field or ultrasonic waves, within not less than 0.5 min. and not more than 36 hr. after the start of injection, can raise the temperature of a tumorous tissue in the close vicinity of the magnetic vesicular particles.

The manufacturing method of the preparation containing magnetic vesicular particles of the invention comprises mixing a lipid membrane constituents and supercritical carbon dioxide and adding a dispersion of magnetic microparticles having particulate gold attached onto the surface with an organic compound which has a linking group and is chemically bonded to the particulate gold, followed by discharge of carbon dioxide, whereby the magnetic microparticles are covered with a lipid membrane to form magnetic vesicular particle(s).

The foregoing magnetic microparticles having particulate gold attached to the surface are formed preferably by dispersing magnetic microparticles in a gold ion- or gold complex-containing solution or adding the gold ion- or gold complex-containing solution to a dispersion of the particulate gold dispersed in a dispersing medium, followed by exposure of the obtained solution to ultrasonic or ionizing radiation.

The magnetic microparticles having particulate gold which is attached to the microparticle surface and an organic compound which contains a linking group and is chemically bonded to the particulate gold, is formed preferably by dispersing magnetic microparticles having particulate gold attached to the microparticle surface, in a solution containing an organic compound with a linking group, or by adding the organic compound with a linking group to a solution containing the magnetic microparticles having particulate gold attached to the microparticle surface, followed by being mixed with stirring.

The linking group is preferably chosen from the group consisting of mercapto group, dithio group, sulfo group, thiocarboxy group, dithiocarboxy group, amino group and hydroxyl group.

The diagnostic therapeutic system of this invention is a system for performing diagnosis of a tumor site of an examinee and therapy thereof, using the above-described preparation containing magnetic vesicular particles. The diagnostic therapeutic system comprising:

> an automatic injection device for automatically injecting the preparation containing magnetic vesicular particles into an examinee,
> a diagnosis device provided with a first exposure section to subject the preparation-injected examinee to a first exposure to an ultrasonic, an electromagnetic wave or an X-ray, and an imaging section to scan a tumor site in which the magnetic vesicular particles have been accumulated by the first exposure,
> a therapy device provided with a second exposure section to subject the magnetic vesicular particle-accumulated tumor site to a second exposure to an alternating magnetic field or an ultrasonic, and a temperature measurement section to measure the temperature of the tumor site and that of a normal site near the tumor site during the second exposure to an alternating magnetic field or an ultrasonic, and
> a control device which is connected to each of the automatic injection device, the diagnosis device and the therapy device through a network, controls the operation of each of these devices and performs control among these devices.

The above-described temperature measuring section preferably conducts temperature measurement which is non-invasive for the examinee and the non-invasive temperature measurement calculates the temperature from a vertical relaxation time by a signal intensity method, a proton chemical shift by a phase method or a diffusion coefficient by a diffusion image method, each of which uses a nuclear magnetic resonance imaging apparatus, or from values obtained in microwave radiometry using plural frequencies.

It is preferred that the temperature measurement section measures the temperature of a tumor site and that of a normal site near the tumor site and successively transmits measurement results to a control device, and when the control device confirms from the received measurement results that the tumor site has risen to a prescribed temperature, the control device transmits an instruction to stop the exposure to the alternating magnetic field or the ultrasonic to the second exposure section, thereby controlling therapy.

It is also preferred that the second is so controlled that the second exposure section exposes the magnetic vesicular particle-accumulated tumor site to an alternating magnetic field or an ultrasonic, while the first exposure section exposes the tumor site to an ultrasonic, an electromagnetic wave or an X-ray and the imaging section scans the magnetic vesicular particle-accumulated tumor site to confirm the location of the tumor site, whereby therapy is performed with confirming the tumor site.

**EFFECT OF THE INVENTION**

The magnetic vesicular particles contained in the preparation of this invention enable selective delivery to a disease site or a tumor focus (targeting capability). Accordingly, the preparation is not only applicable to a therapeutic agent, a contrast medium and a drug transporting carrier but is also usable in thermotherapy employing heat-generation, and further usable in diagnosis and therapy of cancer by combinations of the foregoing.

In the magnetic vesicular particles of this invention, binding between magnetic microparticles and the lipid membrane is enhanced by allowing particulate gold and the organic compound therebetween, resulting in enhanced stability as a composite particle and leading to a pharmaceutical preparation with enhanced stability.

In the diagnostic therapeutic system of this invention, examination, diagnosis and therapy of a disease site or a tumor focus can be conducted as a single system. Accordingly, diagnosis and therapy which have conventionally been conducted separately, can be done concurrently or continuously, thereby lessening the burden given to the patient.

## BRIEF EXPLANATION OF THE DRAWINGS

FIG. 1 illustrates a magnetic vesicular particle contained in the preparation of the invention, in which a single magnetic microparticle is enclosed within the grain.

FIG. 2 also illustrates a preferred embodiment of the magnetic vesicular particle.

FIG. 3 illustrates an embodiment of a diagnostic therapeutic system employing a preparation containing magnetic vesicular particles.

## EXPLANATION OF NUMERAL

1: magnetic vesicular particle
2: magnetic particle
3: gold particle
4: organic compound
5: lipid membrane
6: physiologically functional material
7: antitumor-active material
8: linking material
10: diagnostic therapeutic system
20: automatic injection device
30: diagnostic device
32: first exposure section
34: imaging section
40: therapeutic device
42: second exposure section
44: temperature measuring section
50: control device
60: network

## PREFERRED EMBODIMENTS OF THE INVENTION

The preparation of this invention contains magnetic vesicular particles (hereinafter, also denoted simply as vesicular particles). The preparation optionally contains auxiliary agents.

The magnetic vesicular particle is comprised of one or more magnetic microparticles covered with a lipid membrane. On the magnetic microparticle surface is attached particulate gold, to which an organic compound is chemically bonded via a linking group. The linking group of the organic group, which exists at the terminal end of the molecule or within the molecule, is not specifically limited, if it has affinity for gold. Examples of such a linking group include mercapto group (-SH), dithio group (-S-S-), sulfo group ($-SO_3H$), thiocarboxy group [-C(=O)SH], dithiocarboxy group [-C(=S)SH], amino group ($-NH_2$) and hydroxyl group (-OH). Of these linking groups, mercapto group, dithio group, sulfo group, thiocarboxy group and dithiocarboxy group are preferred, and mercapto group is more preferred.

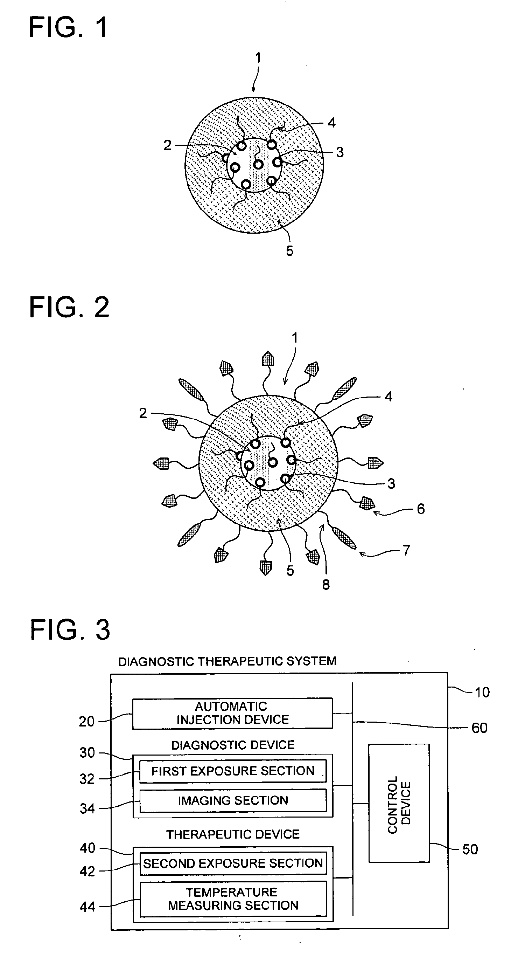

In one preferred embodiment of the magnetic vesicular particles, for example, as shown in FIG. 1, particulate gold 3 is attached onto the surface of magnetic microparticle 2. The magnetic microparticle 2 having the particulate gold to which organic compound 4 having a linking group such as a mercapto group is chemically bonded, is covered with a lipid membrane to form a nano-level structure. FIG. 1 shows an embodiment of magnetic vesicular particle 1 in which a single magnetic microparticle is covered with lipid membrane, but the magnetic vesicular particle is not limited to this. Thus, the magnetic vesicular particle may be formed of two or more magnetic microparticles, which may be covered with lipid membrane.

In one aspect of the invention, the magnetic vesicular particles satisfy the following equation:

$$0.02 \leq R/(r \times 100) \leq 0.30$$

wherein R represents an average particle size of the magnetic vesicular particles and r represents an average particle size of the magnetic microparticles covered with lipid membrane.

In the magnetic vesicular particle 1 used in this invention, as shown in FIG. 2, particulate gold 3 is attached to the surface of magnetic microparticle 2. The magnetic microparticle 2 having the particulate gold to which organic compound 4 having a linking group such as a mercapto group is chemically bonded, is covered with a lipid membrane. The surface of the vesicular particle is preferably bonded via linkage material 8 to physiologically functional material 6 such as an antibody or antitumor-active material 7. Although FIG. 2 shows an embodiment of the magnetic vesicular particle in which plural magnetic microparticles are covered with lipid membrane, a single magnetic microparticle may be covered with lipid membrane. FIG. 2 shows an embodiment of magnetic vesicular particle 1 in which a single magnetic microparticle is covered with lipid membrane, but the magnetic vesicular particle is not limited to this. Thus, the magnetic vesicular particle may be formed of two or more magnetic microparticles, which may be covered with lipid membrane. In FIG. 2, a physiologically functional material and an antitumor-active material are bonded but there may also be bonded at least one physiologically active material selected from physiologically functional materials, additionally stabilizing material, medicinally active material, medicinally active chelate material, antitumor-active material, immunopotentiation material, cell fusion material and gene transfer mediator material to be described later. As a result of bonding to physiologically active material, the magnetic vesicular particles are delivered to the tumor site not only for detection but also exerts a specific effect on tumorous tissue. Accordingly, it is usable as a contrast medium exhibiting superior detectability for tumorous tissue and in thermotherapy using energy exposure such as exposure to an alternating magnetic field or exposure to a ultrasonic wave, it raises the temperature of tumorous tissue in the close vicinity of the magnetic vesicular particles and is also usable as a therapeutic agent which is capable of allowing physiologically active material to act onto the tumorous tissue. FIG. 1 and 2, each illustrates a magnetic vesicular particle contained in the preparation of this invention but specific embodiments are by no means limited to these.

The vesicular particles which include magnetic microparticles within lipid membrane is manufactured by the process comprising at least the following steps:

(i) magnetic microparticles is dispersed in a solution containing gold ions or a gold complex, or solution containing gold ions or a gold complex is added to a dispersion of magnetic microparticles dispersed in a dispersing medium, subsequently, the obtained solution is exposed to ultrasonic or ionizing radiation to form magnetic microparticles having particulate gold attached to the magnetic microparticle surface;
(ii) the magnetic microparticles having particulate gold attached onto the magnetic microparticle surface is dispersed in a solvent which is not reactive with a linking group of an organic compound, and further thereto, a solution containing the organic compound having the linking group is added with stirring to form magnetic microparticles having the particulate gold attached onto the microparticle surface, and the particulate gold being chemically bonded to the organic compound having the linking group; and
(iii) the thus formed magnetic microparticles are subjected to a supercritical carbon dioxide process to allow the magnetic microparticles to be enclosed within a lipid membrane.

In the specification, "cancer" refers to a malignant tumor and it is also referred to simply as "tumor". The expression, being enclosed within lipid membrane or liposome membrane means being included in the liposome membrane or liposome and associated with the lipid membrane, or existing in a water phase (internal water phase) enclosed in the interior of the lipid membrane.

Magnetic microparticles usable in this invention can use, as a main component, any one of magnetite, $Fe_2O_3$, $Fe_3O_4$, mixed ferrite, and other iron-containing compounds including organic ferromagnetic material. Of these, ferrite, $Fe_3O_4$ exhibiting a maximum force, which is superior in magnetic responsibility, is specifically preferred. There was developed a technique in which nano-sized microparticles of ferrite (solid solution of $Fe_3O_4$ and $\delta$-$Fe_2O_3$) exhibiting superior magnetic characteristics were synthesized by a controlled precipitation method under mild conditions of a temperature of 4 to 25 °C and a neutral pH, as described in JP-A No. 2002-128523. The preparation of this invention employs such mixed ferrite microparticles as suitable magnetic microparticles. Magnetic microparticles having the foregoing ferrite as a core can further contain various metal elements such as Zn, Co and Ni to control magnetic characteristics.

The average particle size of the magnetic microparticles is usually from 5 to 30 nm, preferably from 5 to 25 nm, and more preferably from 5 to 20 nm.

The magnetic vesicular particles 1, each contains at least one magnetic microparticle as a ferrite core. The number of magnetic microparticles is variable depending on the average size of magnetic microparticles, the average size of magnetic vesicular particles and magnetic characteristics required as the preparation of this invention, therefore, the number of magnetic microparticles is optimally adjusted.

The magnetic microparticles can employ commercially available ones and can also manufactured employing commonly known methods. For instance, magnetic microparticles may be manufactured according to the method described in U.S. Patent No. 5,514,394 and "Nature Materials" vol. 3, No. 12, page 891-895 (2004).

Regarding the average grain size of magnetic vesicular particles having at least one magnetic microparticle, it is necessary to take sizing of the grain size into account to maintain passive targeting capability. Japanese Patent No. 2619037 describes that removal of liposomes of particle sizes of 3000 nm or more can avoid embolization of pulmonary capillaries. However, liposomes of sizes of 150 to 3000 nm are not always antitumorous. It therefore needs to optimally design the particle size according to the objective of the contrast medium. In this invention, the average grain size of magnetic vesicular particles is usually from 50 to 200 nm, preferably from 80 to 200 nm, and from 50 to 150 nm. For example, to achieve selective delivery to the tumor site, the average grain size from 80 to 150 nm is specifically preferred. Making the grain size uniform within 100 to 200 nm (preferably 110 to 130 nm) enables to allow magnetic vesicular particles to be selectively concentrated to cancerous tissue (EPR effect). Pores of neovascular walls existing in solid cancerous tissue are extraordinarily large, as compared to the pore size of capillary wall fenestra of normal tissue, 30 to 80 nm and even molecules having a size of ca. 100 nm to ca. 200 nm leak from the vascular wall. The EPR effect is due to the fact that a neovascular wall existing in cancerous tissue exhibits higher permeability than a microvascular wall, so that blood retention capability have to be enhanced. The magnetic vesicular particles are not so large and are difficult to become a target of capture by a reticulated endothelial cell.

In cases when the preparation of this invention is used as a contrast medium, the average size of magnetic vesicular particles which falls within the foregoing range results in enhanced detection capability of cancerous tissue. On the other hand, in cases when the preparation of this invention is used as a therapeutic agent for use in thermotherapy for tumor and when an average grain size falls within this range, the temperature of tumorous tissue close to the magnetic vesicular particles is limitedly raised upon energy exposure, substantially without affecting normal tissue. When the energy exposure is exposure to an alternating magnetic field, the average size of magnetic microparticles is preferably not less than 10 nm in terms of rotatability of the magnetic microparticles under the alternating magnetic field. An average particle size of less than 10 nm results in poor rotation of the magnetic microparticles, leading to a deteriorated temperature increasing efficiency. Specifically, the average particle size is usually from 10 to 30 nm, preferably from 10 to 25 nm, and more preferably from 10 to 20 nm.

Magnetic microparticles are desirably as large as possible from the viewpoint of contrast performance (specifically, T2 relaxation time) of a contrast medium for use in the nuclear magnetic resonance imaging or simply magnetic resonance imaging (MRI) or from the viewpoint of function as a heat-generating element of thermotherapy, with considering that the particle size and the magnetic moment are dependent on each other, affecting their effects. On the other hand, the grain size of magnetic vesicular particles is limited to be not more than a prescribed size from various biological characteristics, specifically, target directionality. Accordingly, as a preferable range of the average size to find a compromise point between both parameters, the magnetic vesicular particles are required to satisfy the following equation, indicating the relationship between the particle size of magnetic microparticles and the grain size of magnetic vesicular particles:

$$0.02 \leq R/(r \times 100) \leq 0.30,$$

preferably, $0.03 \leq R/(r \times 100) \leq 0.30$, and
more preferably, $0.03 \leq R/(r \times 100) \leq 0.24$,
wherein R represents the average grain size of magnetic vesicular particles and r represents the average particle size of magnetic microparticles included in the magnetic vesicular particles.

When the magnetic vesicular particles satisfy the foregoing equation, sizes of constituent magnetic microparticles and the size of the whole particles are guaranteed to fall within the suitable range. Thereby, the magnetic vesicular particles can be delivered specifically to the tumor site. As a result, the magnetic vesicular particles are usable as a contrast medium capable of enhancing detection capability or usable as a therapeutic agent capable of limitedly raising the temperature of tumorous tissue in the close vicinity of the magnetic vesicular particles in thermotherapy accompanying with energy exposure such as exposure to an alternating magnetic field or exposure to a ultrasonic wave.

The organic compound chemically bonded to magnetic microparticles is one which has in its molecule at least two binding groups (a) selected from hydroxyl group (-OH), carboxyl group (-COOH), carbamoyl group ($-CONH_2$), amino group ($-NH_2$), mercapto group (-SH), sulfo group ($-SO_3H$), dithio group (-SS-), thiocarboxyl group [-C(S)OH or -C(O)SH] and dithicarboxyl group (-CSSH). Herein, the expression, chemically bonded means being bonded through chemical bond. In general, the chemical bond is classified to covalent bond, ionic bond, metallic bond and coordinate bond (including chelate bond). The organic compound may be bonded to the magnetic microparticles through any of the foregoing chemical bonds. These groups (a) are each chemically bonded to the magnetic microparticle surface, whereby polyvalent bonding effects of the organic compound are displayed. Thus, the organic compound is bonded to the magnetic microparticles through at least two binding groups, forming strong linkage to the magnetic microparticles. Such a poly-binding organic compound allows plural magnetic microparticles to be connected to each other and is further bound to lipid membrane 4 covering magnetic microparticle(s) 2, playing the role as a connector. As a result, magnetic vesicular particles are entirely structurally stabilized. Magnetic microparticles can stably be enclosed within lipid membrane by the existence of organic compound 3. In one case, the organic compound bonding to the magnetic microparticle may be linked at the other end to physiologically active material or medicinally effective material. Lipophilic physiologically active material or medicinally effective material which is delivered by magnetic vesicular particles as a carrier is expected to preferably interact with lipid membrane 4 covering magnetic microparticle(s) 2.

Magnetic microparticle 2 used in the preparation of this invention is one having particulate gold 3 adhered onto the surface. The expression, having particulate gold attached to the surface means that the particulate gold is held on the surface of the magnetic microparticle, like being connected thereto. Its bonding manner and bonding strength are described in WO 2004/083124, described earlier.

The relation ship of particle size between the magnetic microparticle and the particulate gold preferably satisfies the following equation:

$$0.01 \leq r_2/r_1 \leq 10 \text{ (more preferably, } 0.05 \leq r_2/r_1 \leq 5)$$

wherein $r_1$ is an average particle size of magnetic microparticles and $r_2$ is the average particle size of the particulate gold.

An average particle size of the particulate gold, falling within the foregoing range results in enhanced bonding to the magnetic microparticle and superior balance in size between magnetic microparticle and particulate gold, whereby, the magnetic vesicular particles can be delivered specifically to the tumor site. As a result, the magnetic vesicular particles are usable as a contrast medium capable of enhancing detection capability or usable as a therapeutic agent capable of limitedly raising the temperature of tumorous tissue in the close vicinity of the magnetic vesicular particles in thermotherapy accompanying with energy exposure such as exposure to an alternating magnetic field or exposure to ultrasonic waves.

There will be described a method of allowing particulate gold to be attached to the magnetic microparticle surface, as below.

A dispersion of magnetic microparticles co-existing with gold ions or a gold complex is prepared in such a manner that the magnetic microparticles are dispersed in a solution containing gold ions of a gold complex, or a solution containing gold ions of a gold complex is added to a dispersion of magnetic microparticles. The thus obtained dispersion is subjected to exposure to ultrasonic or ionizing radiation, whereby magnetic microparticles having gold atoms or gold nano-particles attached to their surface, can be obtained. It is contemplated that gold ions or a gold complex contained in the solution are reduced by energies of exposed ultrasonic or ionizing radiation to deposit simple substance of gold or elemental gold.

The foregoing solution containing gold ions or a gold complex is an aqueous or alcoholic gold ion-containing solution. The solution can be prepared by dissolving an appropriate compound providing a gold ion in water, a water-containing alcohol, alcohols such as methanol, ethanol, and isopropanol or mineral acids such as hydrochloric acid, sulfuric acid, and nitric acid. Compounds used for preparing such a gold ion-containing solution include, for example, a nitrate, chloride, acetate, citrate, sulfate, carbonate, oxide and hydroxide of gold. Specifically, an aqueous solution of a chloride or nitrate which is easily dissolved in water and low-priced, and a hydrochloric acid or nitric acid solution of an oxide are preferred. The prepared gold ion concentration is not specifically limited but the gold ion concentration of a gold ion-containing solution is preferably from 1 μM to 1 M, and more preferably 0.1 mM to 10 mM. An excessively high gold ion concentration of more than 1 M results in formation of particulate gold with excessively large sizes or formation of excessively large amounts of particulate gold. A gold ion concentration of less than 1 μM often results in no deposition of particulate gold on the magnetic microparticle surface.

As a gold complex containing solution are cited an aqueous solution and a water-containing alcohol or alcohol solution, each containing a compound in which the foregoing gold ion is coordinated with an appropriate ligand. A ligand to form a complex is not specifically limited so long as it has a non-covalent electron pair. For instance, it can optimally be selected from unidendate ligands such as a halide ion, cyanide ion and ammonia, and compounds capable of coordinating at a dendate of two or more, such as ethylenediamine, bipyridyl, acetylacetonato ion, triethylenediamine, tetraethylenediamine and ethylenediaminetetraacetic acid ion.

To the foregoing solution containing gold ions or a gold complex, there may optionally be incorporated various additives to control the reduction reaction rate of metal ions or the particle size of particulate gold. Examples of such an additive include a water-soluble polymeric compound such as polyvinyl alcohol (PVA), a surfactant, alcohols, ethers such as tetrahydrofuran, polyols such as alkylene glycol, carboxylic acids such as acetic acid and ketones such as acetone.

The amount of magnetic microparticles dispersed in a solution containing gold ions or a gold complex is preferably 0.001% to 50% by weight based on the solution and more preferably 0.1% to 10% by weight. A gold ion content falling within the foregoing range results in formation of microparticles satisfying the relationship of particle size between the magnetic microparticle and the particulate gold, as described earlier.

Exposure to ultrasonic or ionizing radiation can be conducted under such a condition that gold ions or a gold complex are/is reduced to deposit gold atoms. Exposure to ultrasonic is conducted preferably at a frequency of 10 kHz to 10 MHz and a power of 1 W or more, and more preferably in an atmosphere of inert gas such as argon. Any one of a direct-ionizing radiation or an indirect-ionizing radiation is applicable as an ionizing radiation within a range not inhibiting effects of this invention. Examples of such a direct-ionizing radiation include charged particle rays of an electron, proton, $\alpha$ particle and the like. Examples of an indirect-ionizing radiation include non-charged particle rays, such as $\gamma$- ray, X-ray and neutron ray. The wavelength of an ionizing radiation is usually less than 1 mm and preferably not more than 0.1 nm to form fine noble metal particles of a uniform size for a short period of time. Exposure to ionizing radiation is usually at least 1 J/kg as absorption dose, and preferably 1 to 1,000,000 J/kg. Exposure to $\gamma$-ray as ionizing radiation is conducted preferably at a dose of at least 1 Gy.

Exposure to ionizing radiation is conducted preferably with stirring the solution to maintain a dispersed state of magnetic microparticles. Exposure to ultrasonic may be performed without stirring as the exposure to ultrasonic itself has a stirring effect. Exposure to ionizing radiation may be conducted in combination with exposure to ultrasonic. Temperature conditions during exposure are not specifically limited but exposure is performed preferably at a temperature of 0 to 100 °C.

Exposure to ultrasonic and/or ionizing radiation is performed with dispersing magnetic microparticles in a solution containing gold ions or a gold complex, whereby particulate gold is attached to the magnetic microparticle surface. As described in WO 2004/083124, a magnetic of microparticle-forming constituents and a solution containing gold ions or a gold complex may be exposed to ultrasonic and/or ionizing radiation, whereby magnetic microparticles are formed and particulate gold is allowed to attach to the magnetic microparticle surface. Alternatively, a solution containing gold ions or a gold complex is exposed to ultrasonic and/or ionizing radiation and the formed particulate gold is mixed with magnetic microparticles. Organic compound 4

In one aspect of the magnetic microparticles of this invention, as shown in FIG. 1, particulate gold 3 is attached to the surface of magnetic microparticle 2 and organic compound 4 having a linking group is bonded to the particulate gold. Specifically, a mercapto group is preferred as the linking group. As is well known, reaction of a mercapto group with gold extremely rapidly proceeds through spontaneous bond formation and the formed Au-S bonding is strong due to a self-assembling bond. Accordingly, bonding of a compound having a mercapto group to the particulate gold can be performed by a simple procedure under relatively mild conditions.

Interaction of the moiety of the organic compound 4 except for a mercapto group bonding to the particulate gold (gold nano-particle) and lipid membrane constituent can bring about formation of an organic-inorganic hybrid microparticle in which the inorganic material portion and the organic material portion are not easily broken. When energy exposure, such as exposure to an alternating magnetic field or exposure to ultrasonic causes rotation, stabilities such as maintenance of bonding between the inorganic material portion and organic material portion and integration of the overall composite particle structure become essential. Accordingly, the preparation of this invention which contains composite magnetic particles including bonding between gold and magnetic iron oxide microparticles (as described in patent document 3) and AU-S bonding, can be stably used over a long term as an imaging agent (e.g., contrast medium) or an agent used for thermotherapy.

An organic compound (4) containing plural mercapto groups can also be optimally used. Such a poly-binding organic compound (4) allows plural magnetic microparticles (2) to be connected to each other and is further bound to lipid membrane (5) covering magnetic microparticle(s) 2, playing the role as a connector. As a result, magnetic vesicular particles are entirely structurally stabilized. Magnetic microparticles can be stably enclosed within a lipid membrane by the existence of the organic compound (4).

Any organic compound containing at least one mercapto group can be used as mercapto group-containing organic compound (4). To enhance interaction with lipid membrane 5 are preferred amphipathic, medium or long chain organic compounds containing, as an organic group of the organic compound (4), alkyl, aralkyl, alkoxy, aryl, alkyleneoxy, an aliphatic hydrocarbon group or polyoxyalkylene group, which may be substituted. The organic compound (4) preferably plays a role in combining magnetic microparticles and the lipid membrane by allowing the organic group and the lipid membrane to interact with each other via various types of bonding, such as a covalent bond, ionic bond, hydrophobic bond, and a hydrogen bond. From such a point of view, the organic group is preferably a hydrophobic, medium or long chain alkyl group or dissociative group which is capable of interacting with the phospholipid of the lipid membrane.

Therefore, an organic compound (4) is preferred which contains a mercapto group including a straight chain aliphatic hydrocarbon group or a polyalkyleneoxy group. The organic compound preferably plays a role in combining magnetic microparticles and the lipid membrane by allowing the organic group (b) and the lipid membrane to interact with each other via various types of bonding, such as a covalent bond, ionic bond, hydrophobic bond, and hydrogen bond. From such a point of view, the organic group (b) is preferably a hydrophobic, medium to a long chain alkyl group or dissociative group which is capable of interacting with the phospholipid of the lipid membrane. Accordingly, a mercapto group-containing organic compound (4) including a straight chain aliphatic hydrocarbon group or a polyalkyleneoxy group of 3 to 30 carbon atoms is preferred.

The mercapto group-containing organic compound (4) may contain other substituents capable of bonding with particulate gold or magnetic microparticles, within a range not to inhibit the object of this invention. Examples of such a substituent resulting in a cross-linking effect include a hydroxy group, carboxy group, carbamoyl group, amino group, sulfo group, dithio group, thiocarboxy or dithiocarboxy group, imino group, ether group, carboxylic acid residue (carboxylate), phosphoric acid residue and dithio residue.

In one case, the organic compound (4) bonding to the particulate gold (3) may be linked at the other end to a physiologically active material, medicinally effective material or a molecule recognition material. Lipophilic physiologically active material or medicinally effective material which is delivered by magnetic vesicular particles as a carrier is expected to preferably interact with lipid membrane 4 covering magnetic microparticle(s) 2. There are commercially available, for instance, various active compounds, peptides, proteins and alkanethiol or alkanedisulfide compounds containing a carboxy, amino or hydroxy group as an end group to introduce a molecule recognition site. Specific examples thereof include 6-amino-1-hexanethiol, 8-amino-1-octanethiol, 11-amino-1-undecanethiol, 5-carboxy-1-pentanethiol, 7-carboxy-1-heptanethiol, 10-carboxy-1-decanethiol, 6-hydroxy-1-octanethiol, 11-hydroxy-1-undecanethiol, 10-carboxydecyldisulfide, 7-carboxyheptyldisulfide, $CH_3(CH_2)_{17}SH$, $CH_3(OCH_2CH_2)_6SH$ and $CH_3(OCH_2CH_2)_{10}SH$.

Allowing an organic compound (4) containing a linking group (preferably, a mercapto group) to be chemically bonded to the particulate gold (3) on the surface of the magnetic microparticle can be achieved in such a manner that magnetic microparticles having particulate gold attached to the particle surface in a solvent which is non-reactive to the organic compound (4) and further thereto, a solution containing the organic compound (4) is added with stirring. Alternatively, magnetic microparticles having particulate gold attached to the particle surface are added to a solution containing an organic compound containing a linking group, while stirring. The addition amount of an organic compound containing a linking group can be optimally adjusted, depending on the molecular weight of the organic compound, the amount or surface area of the particulate gold attached to magnetic microparticles and an extent of allowing the particulate gold to be attached.

The organic compound (4) containing a linking group may be bonded to particulate gold by using the above-described organic compound containing an organic group (A). Alternatively, after an organic compound having a basic skeleton of a compound containing a linking group is allowed to bond to the particulate gold and then, the organic group (A) may be bonded to the basic skeleton.

The thus prepared magnetic microparticles to which an organic compound is chemically bonded, are obtained in the form of a dispersion. When using the thus obtained magnetic microparticles in the manufacture of magnetic vesicular particles, it is preferred that the magnetic microparticles are refined by the prescribed method and dispersed in an aqueous medium. As an aqueous medium is employed water such as distilled water, official water for injection or pure water, physiological saline, various buffer solutions and an aqueous salt-containing solution.

Lipid membrane 5

In the constitution of the magnetic vesicular particle (1), magnetic microparticle (2) having particulate gold (3) which are bonded to organic compound (4) are covered with a lipid membrane (as shown in FIG. 1 and 2). Covering magnetic microparticles with a lipid membrane allows the particles to be dispersed in an aqueous medium and makes it feasible to form a magnetic vesicular particle-containing preparation exhibiting superior dispersion stability. Magnetic microparticles exhibiting a relatively high residual magnetism often cause magnetic coagulation, forming precipitates in medium. The lipid membrane is inherently biocompatible and exhibits enhanced affinity to tissue, and specifically, directionality to hydrophobic tissue is provided to magnetic microparticles. Physiologically active material, medicinally effective material or the like can be advantageously added through designation of lipid membrane constituents, modification of the membrane surface and the like.

Lipid membrane covering magnetic microparticles is generally lipid multi-layer membrane, preferably a multi-layer membrane formed of an amphiphilic molecule having a medium or long chain aliphatic acid residue or a medium or long chain alkyl group and a hydrophilic group. Phospholipid and/or glycolipid are preferably used as such a lipid membrane constituent. A vesicle constituted of lipid bilayer (mainly composed of phospholipid) is generally referred to as "liposome". Accordingly, lipid membrane covering the magnetic microparticle is liposome and the surface charge of the liposome is desirably positive.

Representative examples of a phospholipid include phosphatidylcholine, phosphatidylserine, phosphatidylethanolamine, phosphatidylglycerol, phosphatidylinositol, phosphatidic acid, cardiolipin and sphingomyelin. There are also usable phospholipids derived from plants and animals such as egg yolk or soybeans and their hydrogenation products or hydroxide derivatives, so-called semi-synthetic phospholipids. Fatty acids constituting a phospholipid are not specifically limited, and saturated and unsaturated fatty acids are usable.

Specific examples of neutral phospholipid include dipalmitoylphosphatidylcholine (DPPC), distearoyl-phosphatidylcholine (DSPC), dimyristoylphosphatidylcholine (DMPC), dioleylphosphatidylcholine (DOPE), dimyristoylphosphatidylethanolamine, dipalmitolphosphatidylethanolamine, and distearoylphosphatidylethanolamine.

In this invention, the lipid membrane preferably forms a liposome. Thus, the magnetic vesicular particles of this invention preferably are liposomes including one or more magnetic microparticles within lipid membrane vesicles. The liposomes preferably exhibit a cationic surface; in other words, liposome vesicles preferably have the cationic surface. To make the liposome surface charge positive, it is preferred to use, together with the foregoing neutral phospholipid, at least one selected from a cationic phospholipid, a cationic lipid and along chain cationic compound compatible with a phospholipid. Cationic surface charge of liposome membrane enables specific introduction of magnetic vesicular particles contained in the preparation into a negatively charged tumor cell.

Examples of cationic phospholipid include an eater of phosphatidic acid and aminoalcohol, such as an ester of dipalmotoylphosphatidic acid (DPPA) or distearoylphosphatidic acid, and hydroxyethylenediamine. Examples of cationic lipids usable in the invention include 1,2-dioleoyloxy-3-(trimethylammonium)propane (DOTAP), N,N-dioctadecylamidoglycylspermine (DOGS), dimethyloctadecylammonium bromide (DDAB), N-[1-(2,3-dioleyloxy)propyl]-N,N,N-trimethylammonium chloride (DOTMA), 2,3-dioleyloxy-N-[2(spermine-carboxamido)ethyl]-N,N-dimethyl-1-propaneaminiumtrifluoroacetate (DOSPA) and N-[1-(2,3-dimyristyloxy)propyl]-N,N-dimethyl-N-(2-hydroxyethyl)ammonium bromide (DMRIE). Examples of a long chain cationic compound include at least 10 carbon atoms containing onium salts such as ammonium salt or phosphonium salt.

Examples of glyceroglycolipids include glycerolipids such as digalactosyldiglyceride and digalactosyldiglyceride sulfuric acid ester; sphingoglycolipids such as galactosylceramide, galactosylceramide sulfuric acid ester, lactosylceramide, ganglioside G7, ganglioside G6 and ganglioside G4.

To combine a physiologically active material with the foregoing phospholipid, a functional group capable of being bonded to a physiologically active material may be introduced within a range not deviating from the object of this invention. In addition to the foregoing lipid, other material may optionally be incorporated as a liposome membrane constituent. Examples thereof include glycols such as ethylene glycol and propylene glycol, sterols acting as a membrane stabilizer such as cholesterol, dihydrocholesterol and cholesterol ester. Sterol derivatives are also effective for stabilization of liposome, as described in JP-A No. 5-245357. Of these, cholesterol is specifically preferred.

Cholesterol is used preferably in an amount of 0.05 to 1.5 parts by weight, based on 1 part by weight of phospholipid, more preferably 0.2 to 1 part by weight, and still more preferably 0.3 to 1.5 parts by weight. An amount of less than 0.05 parts by weight does not achieve stabilization by the sterol to enhance dispersibility of mixed lipids, while an amount of more than 1.5 parts by weight inhibits liposome formation or results in unstable formation thereof.

Other additive compounds include, for example, phosphoric acid dialkyl esters as negative-charged material, e.g., diacetyl phosphate, and aliphatic amines as a compound providing a negative charge, such as stearylamine.

In this invention, polyethylene glycol (hereinafter, also denoted simply as PEG) can be used as one constituent for liposome covering magnetic microparticles. Thus, attachment of PEG to the liposome can provide a role as "linkage material" as described later or a new function to the liposome. For example, such a PEG-modified liposome can be expected to have an effect of having a hydrophilic tendency, becoming less recognizable from an immune system or increasing blood stability. Specifically, since lipid components easily accumulate in liver, the use of a liposome containing no PEG or trace amounts of PEG is desired. In the case of integrating other organs, the liposome becomes a state of being stealthy by introduction of a PEG, becoming difficult to be gathered in the liver, therefore, the use of a PEG-modified liposome is recommended. Introduction of a PEG forms a hydration sphere, thereby stabilizing the liposome and enhancing blood retention. Functions can be adjusted by changing a length of oxyethylene units of a PEG and its introducing ratio. Polyethylene glycol having 10 to 3500 (preferably, 100 to 2000) oxyethylene units is preferred as PEG. A PEG is preferably contained in an amount of 0.1% to 30% by weight, and more preferably 1% to 15% by weight, based on the lipid constituting the liposome.

A cholesterol enclosed in the liposome membrane is capable of functioning as an anchor to introduce a polyalkylene oxide. Specifically, cholesterol contained as a liposome membrane constituent in the membrane may optionally be attached to a polyalkylene oxide group via a linker. A short chain alkylene or oxyalkylene group is used as a linker. JP-A No. 9-3093 discloses novel cholesterol derivatives, in which various functional substances can be efficiently fixed at the top of a polyoxyalkylene chain, which can then be employed as a liposome constituent.

PEG-modification of a liposome can be accomplished using commonly known techniques. For example, a polyethylene glycol (PEG) group linked to an anchor compound (e.g., cholesterol, phospholipid) is mixed with a phospholipid as a membrane constituent to form a liposome and the anchor compound may be allowed to be linked to an activated PEG group. Since the PEG group introduced onto the liposome surface is unreactive to "physiologically active material" to be described later, it is difficult to fix the physiologically active material onto the liposome surface. Instead thereof, PEG, the top of which has been chemically modified is bonded to a phospholipid, which is included as a liposome constituent to prepare liposomes.

In place of polyethylene glycol (PEG), commonly known polyalkylene oxide groups may be introduced, which is represented by general formula: $-(AO)_n-Y$ where AO is an oxyalkylene group having 2 to 4 carbon atoms, n represents a mean addition molar number and is a positive number of 1 to 2000 , and Y is a hydrogen atom, an alkyl group or a functional group.

Examples of an oxyalkylene group (represented by "AO") having 2 to 4 carbon atoms include an oxyethylene group, oxypropylene group, oxytrimethylene group, oxytetramethylene group, oxy-1-ethylethylene group and oxy-1,2-dimethylethylene group.

In the foregoing, n is 1 to 2000, preferably, 10 to 500, and more preferably 20 to 200. When n is 2 or more, plural oxyalkylene groups may be the same or differ. In the latter case, differing oxyalkylene groups may be in a random form or in a block form. To provide hydrophilicity to a polyalkylene oxide group, ethylene oxide alone, as AO is preferably addition-polymerized, in which n is preferably 10 or more. In cases when different alkylene oxides are addition-polymerized, it is desirable that at least 20 mol% (preferably at least 50 mol%) of ethylene oxide is addition-polymerized. To provide lipophilicity to an oxyalkylene group, it is preferred to increase the molarity of alkylene oxide(s) other than ethylene oxide. For example, a liposome containing a block copolymer of polyethylene oxide and polypropylene oxide (or polyethylene oxide-block-polypropylene oxide) is a preferred embodiment of this invention.

The designation Y is a hydrogen atom, an alkyl group or a functional group. The alkyl group includes an aliphatic hydrocarbon group having 1 to 5 carbon atoms, which may be branched. The functional group of the foregoing Y is to attach functional material such as sugar, glycoprotein, antibody, lectin and a cell adhesion factor to the top of a polyalkylene oxide group and examples thereof include an amino group, oxycarbonylimidazole group and N-hydroxysuccinimide.

The polyalkylene oxide group plays the role of linkage material, as described later, similarly to polyethylene glycol. The liposome anchoring a polyalkylene oxide chain, to the top of which the foregoing functional material is bonded, not only exhibits effects due to introduction of a polyalkylene oxide group but also gives full play of functions of the functional material, for example, a function as a recognition element, such as directivity to a specific organ and cancerous tissue directivity.

A phospholipid or cholesterol which contains a polyalkylene oxide group can be used alone or in combinations thereof. The content thereof preferably is 0.001 to 50 mol%, more preferably 0.01 to 25 mol%, and still more preferably 0.1 to 10 mol%, based on the total amount of liposome membrane forming components. A content of less than 0.001 mol% results in reduced expected effects.

Introduction of a polyalkylene oxide chain into liposomes can employ commonly known techniques. Thus, an anchor bonding a polyalkylene oxide (e.g., cholesterol, phospholipid) is mixed with phospholipid as a membrane constituent to form liposomes and an activated polyalkylene oxide may be attached to the anchor. This method needs to perform multistage chemical reaction on the liposome membrane surface after completing formation of liposomes. As a result, an introducing amount of an objective physiologically active material is limited to the lower side and contamination with reaction by-products or impurities is caused, causing further problems such as marked damage of the liposome membrane.

As a preferred manufacturing method replacing this, phospholipid polyalkylene oxide derivative is included in advance in phospholipids as raw material to form liposomes.

For examples, JP-A No. 7-165770 proposed polyethylene oxide (PEO) derivatives of a phosphatidylamine and the like, such as distearoylphosphatidyldiethanolamine polyethyleneoxide (DSPE-PEO). Further, JP-A No. 2002-37883 discloses an extremely purified, polyalkylene oxide-modified phospholipid to prepare a water-soluble polymer-modified liposome exhibiting enhanced blood retentivity. It is also disclosed that the use of a polyalkylene oxide-modified phospholipid having a relatively low monoacyl content in the preparation of liposome leads to superior aging stability of liposome dispersions.

Linkage material 8

Linkage material as a linker allows various physiologically active materials or medicinally effective materials to be bonded in the interior of the magnetic vesicular particle. When a physiologically active material or medicinally effective material having a relatively low molecular weight, as a ligand, is linked to an acceptor, its approach to the ligand is often hindered by steric hindrance due to the magnetic vesicular particle. Such hindrance can be avoided by allowing a linkage material as a spacer having an appropriate length to intervene between the magnetic vesicular particle and a ligand. Preferred examples of a linkage material include a polyethylene glycol chain and a polyalkylene oxide chain, such as ethylene glycol diglycidyl ether (EGDE). A suitable hydrocarbon chain is one which may contain a heteroatom (e.g., oxygen, nitrogen, sulfur, phosphorus). Such a hydrocarbon chain preferably has 2 to 50 carbon atoms (more preferably 3 to 40 carbon atoms, and still more preferably 4 to 30 carbon atoms), which may be substituted by a functional group, an alkyl group or an aryl group. There may be further provided a binding group such as a thio group, epoxy group, amino group, carboxyl group, histidinetagavidine, streptavidin, and biotin. There may be provided oligonucleotide (no. of nucleic acid base: 3-100) or polypeptide (no. of nucleic acid base: 3-50) which is modified with an amino group, carboxyl group or thiol group.

The linkage material may be one member of lipid membrane constituents, as a lipid derivative constituting the lipid membrane.

Physiologically active material

The magnetic vesicular particles are each bonded to physiologically active material via the linkage material (7) or the organic compound (4), as described above. In the case of liposome-magnetic vesicular particles, physiologically active material may be directly bonded onto the lipid membrane surface of the liposome. Alternatively, physiologically active material is bonded to the organic compound (4) and exists in the interior of the lipid membrane. Examples of physiologically active material include physiologically functional material, additively stabilizing material, medicinally active material, medicinally active chelating material, antitumor-active material, immunopotentiating material, cell fusion material, and gene transfer mediating material. It is also feasible that the foregoing physiologically active material is allowed to bond to magnetic vesicular particles and concentrate selectively to the targeted site through a magnetic operation.

Examples of physiologically functional material include physiological material, such as sugar, glycoprotein, aptamer, antibody, antigen, lectin, cytokine, growth factor, adjustment factor, physiologically active peptide, cell adhesion factor or hormone; and material displaying physiological function, such as metabolic material or an alkaloid.

The antibody may be either a polyclonal antibody or a monoclonal antibody. There are exemplified antibodies attached to various kinds of glycoproteins. Examples thereof include CD44, CD54, CD56 and Fas. Further, an antigen which is expressed specifically in cancer cells and an antibody against tumor-related antigens are also desirable. In this regard, antigens are commonly known, such as MN, HER2, MAGE3, VEGF and CEA.

An antibody against "WTI protein", which does not exist in a normal cell but exists mainly in various kinds of cancer cells or a blood cancer cell, is also cited as a preferred antibody. It is proved that a part of the WTI protein (nine aminoacid-WTI peptides) links to HLA molecule existing on the surface of cancer cell, which becomes a marker of the cancer cell. The foregoing antibody can be prepared by the conventional method, using WTI peptide.

The additively stabilizing material is a material capable of stabilizing a structure of magnetic microparticles which are associated by solvention. Examples thereof include polyvinyl alcohol, polyvinyl pyrrolidone, polyethylene glycol, polyalkylene oxide, dextran, cellulose derivatives, muco-plysaccharide, protein, polypeptide, polyaminoacid, and polynucleotide.

The medicinally active material is a compound exhibiting bioactivity or medicinal effectiveness. Examples thereof include antitumor-active material, anti-infective material, antiviral, antibiotic, anti-inflammatory compound, chemotherapeutic agent, circulatory medicine, alimentary drugs, and neural medicine.

The medicinally active chelating material is material detoxification upon chelate formation or stabilizing action upon chelation. Examples thereof include EDTA, DTPA, cyclin, polycarboxylic acid, polyamino acid, porphyrin, and catecholamine.

The antitumor-active material is material exhibiting tumor-shrinkage effects. Examples thereof include antibiotics, plant alkaloids, alkylation agents, antitumor agents cotained in antimetabolite, anti-vascularization medicine and tumor neocrotizing factors. Examples of an anti-vascularization medicine include TNP-470 (AGM-1470, synthesized analog of fungus discharge, named fumagilin, produced by Takeda Chemical Industries Ltd.), and iostatin (Herbert Medical School, Children's Hospital, Surgical Research Lab.), and integlin $\alpha_v\beta_3$ antagonistic drugs (e.g., monoclonal antibody of integlin $\alpha_v\beta_3$, The Scripps Research Institute, LaJolla, CA).

The immunopotentiation material is one exhibiting action to enhance activity of immunocytes including lymphocyte and macrophage. Examples thereof include interferon, Krestin, Picibanil, Lentinan, IFA and OK-432.

Cell fusion material is used in a cell fusion operation and promotes cell fusion. Examples thereof include polyalkylene glycol, aryl polyalkylalkylene glycol, arylpolyalkylene glycol, alkylarylpolyalkylene glycol and their derivatives.

The gene transfer mediating material is one functioning as a carrier for gene transfer and examples thereof include polyalkylene glycol (e.g., polyethylene glycol), polyimine (e.g., spermine, spermidine, pentaethylenehexaamine, polyethyleneimine, protamine sulfate), virus vectors and plasmid vectors.

The combination of a photosensitizer of liposomes including phospholipid exhibiting a transition temperature, used for photodynamic therapy and cancer thermotherapy is feasible and enhanced therapeutic effects are expected. A magnetic vesicular particle preparation employing a liposome which links or encloses a photosensitizing material such as porphyrins, 5-aminolevulinic acid, chlorines and phthalocyanine, is given to a patient, and after an elapse of time and when heated from the outside of body, local thermotherapy is conducted using microwaves or electromagnetic waves.

13

The preparation containing magnetic vesicular particles, according to this invention, may optionally contain, as auxiliary agents, a pharmaceutically allowable buffering agent, stabilizer, antioxidant such as α-tocopherol, viscosity-adjusting agents or chelating agents. These are optimally employed prevent oxidation reduction reaction or alteration such as coagulation or precipitation. One feature of the preparation of this invention is that any organic solvent is not substantially contained in the included lipid membrane or in the internal water phase. Manufacturing of the preparation containing magnetic vesicular particles

Manufacturing methods of the preparation containing magnetic vesicular particles of this invention are not specifically limited if the magnetic microparticle surface bonded to the afore-mentioned organic compound via particulate gold can be covered with lipid membrane. The preparation can also be manufactured employing a conventional shaking method. However, in conventional methods, the phospholipid needs to be dissolved in chlorinated solvents and unremoved chlorinated solvents are feared to remain in the preparation, often causing problems with respect to safety for the human body. On the contrary, the preparation containing magnetic vesicular particles of this invention can be manufactured substantially without using organic solvents such as chlorinated solvents. Thus, the preparation can be made by the method using supercritical carbon dioxide. The expression, substantially without using organic solvents means that the upper limit of the residual organic solvent concentration of the preparation is 10 μg/L. In the following, supercritical carbon dioxide includes subcritical carbon dioxide.

A preferred method of manufacturing the preparation containing magnetic vesicular particles comprises:

the first step in which the lipid membrane constituent described above and liquefied carbon dioxide are mixed within a pressure vessel, after which applying heat and pressure to the interior of the vessel forms supercritical carbon dioxide, and while mixing the lipid membrane constituents and the supercritical carbon dioxide, a dispersion of magnetic microparticles which are chemically bonded to an organic compound via particulate gold (i.e., magnetic microparticles onto which particulate gold is attached and an organic compound having a linking group is linked to the particulate gold through chemical bonding) is further added thereto and mixed, and

the second step in which after obtaining the mixture in the foregoing step, the interior of the pressure vessel is evacuated to discharge carbon dioxide, thereby covering (or enclosing) the magnetic microparticles with lipid membrane to form magnetic vesicular particles, resulting in an aqueous dispersion of magnetic vesicular particles.

The foregoing method preferably further comprises the third step in which the aqueous dispersion of magnetic vesicular particles is subjected to filtration using a filter membrane of a pore size of 100 to 1000 nm.

The respective steps will be further described as below.

In the first stage, while mixing lipid membrane constituents and liquefied carbon dioxide in a pressure vessel, the pressure and the temperature in the interior of the vessel are adjusted so that the carbon dioxide becomes supercritical, and the lipid membrane constituents and the supercritical carbon dioxide are mixed.

In the manufacturing method of this invention, the pressure suitable for carbon dioxide in a supercritical state is from 50 to 500 $kg/cm^2$ but preferably from 100 to 400 $kg/cm^2$. The temperature suitable for the supercritical carbon dioxide is from 25 to 200 °C, preferably from 31 to 100 °C, and more preferably from 35 to 80 °C. It is preferred to maintain the supercritical state by the combination of temperature and pressure within the foregoing range. Stirring conditions are not specifically limited and suitable ones are appropriately chosen.

Subsequently, while mixing lipid membrane constituents and supercritical carbon dioxide, an aqueous dispersion of magnetic microparticles which are chemically bonded to an organic compound via particulate gold, is further added thereto and mixed. The aqueous dispersion may be added all at once or intermittently with maintaining the supercritical state. The magnetic microparticles which are chemically bonded to an organic compound via particulate gold, can be prepared by dispersing magnetic microparticles onto the surface of which particulate gold is attached, in a solution containing an organic compound having a linking group; or by adding an organic compound having a linking group to a dispersion in which magnetic microparticles having particulate gold attached thereto are dispersed, followed by being stirred.

In the second stage, after obtaining the mixture in the foregoing step, the interior of the pressure vessel is evacuated and carbon dioxide is discharged, whereby an aqueous dispersion of magnetic vesicular particles (or vesicles) in which the magnetic microparticles are covered with lipid membrane.

The organic compound which is bonded indirectly to the magnetic microparticles via particulate gold, has the afore-mentioned organic group (A), a part of which is enclosed in the formation of lipid membrane and connected to the lipid membrane. Thus, the magnetic microparticles and the lipid membrane are tightly bonded via the organic compound. Accordingly, stability within the human body or during storage is enhanced and for instance, the magnetic vesicular particles are not degraded even when subjected to energy exposure in thermotherapy, performing efficient thermotherapy. In this stage, one or plural magnetic microparticles are enclosed (or covered) with lipid membrane, whereby an aqueous dispersion of magnetic vesicular particles is obtained. In the third stage, the aqueous dispersion of magnetic vesicular particles, obtained in the second stage, is filtered using a filter membrane having a pore size of 100 to 1000 nm.

Specifically, the aqueous dispersion is passed through an extruder installed with a filter having a pore of 100 to 1000 nm, whereby 100-150 nm magnetic vesicular particles can be efficiently made. Thus, adjustment of size and size-distribution of the magnetic vesicular particles can achieve the objective effects of the preparation containing magnetic vesicular particles.

The magnetic vesicular particles of this invention have such an advantage that the dose of magnetic microparticles, in other words, the dosage of lipid can be reduced, compared to magnetic vesicular particles comprised of multilamellar vesicles (also denoted simply as MLV). In the conventional manufacturing method of liposomes, MLV of various sizes and forms often exist in a considerable quantity. Accordingly, an operation such as ultrasonic exposure or passing through a filter having a prescribed pore size a few times was needed to enhance the proportion of unilamellar or several lamellar liposomes. On the contrary, in the method of this invention in which liposomes are formed using supercritical carbon dioxide, liposomes of unilamellar or several-lamellar vesicles can efficiently be produced, whereby an enhanced rate of enclosing a drug in the liposomes can be achieved.

The thus obtained aqueous dispersion of magnetic vesicular particles is filtered using the filtration membrane described above to make a preparation containing magnetic vesicular particles, which may be further subjected to centrifugal separation or ultrafiltration to separate an aqueous medium from the dispersion, whereby concentrated dispersion is obtained.

The preparation containing magnetic vesicular particles of this invention are usable as a medical preparation, i.e., as an imaging agent for used in examination or diagnosis and also as a therapeutic agent. Specifically, it can be used as a contrast medium such as a radiographic contrast medium, a contrast medium for use in MRI (nuclear magnetic resonance imaging method) or an ultrasonic contrast medium, and as a therapeutic agent for cancer or the like, preferably, a therapeutic agent for use in thermotherapy.

Contrast medium (visualizing agent)

The radiographic contrast medium is given to a lumen region such as a vascular tract, a ureter or a uterine tube to be used for examination of a form or stenosis of the lumen. However, conventionally used compounds are promptly discharged from the lumen region without interacting with tissue or disease regions, which is not useful for detailed examination of the tissue or diseased region, specifically such as cancerous tissue. Therefore, an X-ray contrast medium has been desired which can be selectively accumulated in/or onto the targeted tissue or diseased region, thereby giving an image which can be distinguished with clear contrast from the circumference or other regions.

Examination and diagnosis in MRI produce no problem due to radiation exposure and any sectional image of organism can be obtained non-invasively, which is a rapidly spreading image diagnosis technology. To obtain clear images by enhancing contrast, a contrast medium capable of varying the proton relaxation time is employed in MRI. As a contrast medium for use in MRI, gadolinium diethylenetriaminepentaacetate (hereinafter, also denoted simply as Gd-DTPA) is only one contrast-enhancing agent at the present time. The Gd-DTPA contrast medium which has been introduced into a human body is transferred to tissue by the circulating bloodstream. However, the contrast medium itself has no capability of discriminating tissue.

Ultrasonic image diagnostics usually allow ultrasonic having a frequency of 1 to 10 MHz to permeate into the interior of the body of an examinee via a converter, on the basis of ultrasonic waves interacting with the interface of body tissue or body liquid (or humor). An image formed by ultrasonic signals is derived from differential reflection/absorption of ultrasonic in the interface. An ultrasonic contrast medium of microcapsules enclosing gas or bubbles is used in this diagnosis method. However, the amount of enclosed gas or bubbles is not always so large, depending on other factors. Accordingly, a sufficient contrast effect is achieved only by a large amount of dose.

The magnetic vesicular particles which have been introduced into the body as a contrast medium for use in radiography or MRI or as a ultrasonic contrast medium can be selectively accumulated in/or onto the targeted tissue or diseased region, resulting in an image which can be distinguished with clear contrast from the circumference or other regions. Thus, the magnetic vesicular particles of this invention can be selectively accumulated in/or onto the targeted tissue or disease region through their grain sizes or surface charge and can provide the capability of discriminating tissue via physiologically active material attached to the lipid membrane surface. Furthermore, imaging of tumorous tissue can be effectively realized, due to the fact that the ultrasonic propagation in magnetic microparticles is faster than that in a living body (water). Thus, employing such characteristics, the preparation containing magnetic vesicular particles can be used suitably as a contrast medium for use in radiography or MRI, or an ultrasonic contrast medium.

Specifically, within not less than 1 min. and not more than 48 hr., preferably not less than 30 min. and not more than 36 hr. after the preparation containing magnetic vesicular particles of this invention is given into a vein of an examinee, a scan is conducted in a ultrasonic imaging diagnosis apparatus, a nuclear magnetic resonance imaging diagnosis apparatus or an X-ray imaging diagnosis apparatus, thereby achieving enhanced detection of tumorous tissue. The preparation containing magnetic vesicular particles may be directly injected near tumorous tissue of an examinee; within not less than 0.5 min. and not more than 36 hr., preferably not less than 10 min. and not more than 24 hr. after the preparation containing magnetic vesicular particles of this invention is dosed into a vein of an examinee, scan is conducted in a ultrasonic imaging diagnosis apparatus, a nuclear magnetic resonance imaging diagnosis apparatus or an X-ray imaging diagnosis apparatus, thereby achieving enhanced detection of tumorous tissue.

The magnetic vesicular particle-containing preparation of this invention is usable as an imaging agent for examination and diagnosis. Since the preparation bonds or encloses a physiologically active material, medicinally effective material or an antitumor agent inside or outside the lipid membrane, the preparation is also usable as a therapeutic agent for various diseases. The magnetic vesicular particles are accumulated selectively onto focus such as a diseased region or tumorous tissue. Action of a physiologically active material or medicinally effective material is effectively displayed, thereby reducing adverse effects.

Specific application is exemplified below, but application of the preparation of this invention is not limited to this.

Thermotherapy for cancer

Thermotherapy for cancer as well as laser therapy or photodynamic therapy belongs to a category of a non-invasive treatment for cancer. Such cancer thermotherapy, which is a treatment having specific characteristics and superior faces, has not necessarily been adopted in treatment sites for cancer. Thermotherapy has not been employed alone but is employed in combination with radiation or an anticancer drug for enhancing effects of the radiation or anticancer drugs. There may be cited various reasons therefor. Such therapy has not always achieved superior results to other types of treatment. There may be no reason for this therapy to be replaced by other treatments. There is still room for improvement of members, drugs and devices.

The present target of this therapy is locally progressive cancer and recurring cancer which are difficult to be cured in usual treatments. Thermotherapy include warming the whole body (general thermotherapy) and warming the cancer site or its vicinity (local thermotherapy). In general, local thermotherapy is mainly conducted using a device employing microwaves, electromagnetic waves (alternating magnetic fields) or ultrasonic waves to perform localized warming. A system of warming from the outside of the body is most frequently conducted. There is also attempted the method of inserting an instrument into a lumen such as esophagas, rectum, uterus or bile duct and a method inserting several electrode needles into cancerous tissue to perform warming. Effects on cancer are achieved at 41 °C or more, and preferably 42.5 °C or more. Cancer near the body surface can readily be warmed to an intended temperature. Cancer localized deep in the body is often difficult to be sufficiently warmed due to hindrance of fat, air or bone. Introducing the magnetic vesicular particle-containing preparation of this invention into a body to be used as a heating element for thermotherapy can achieve effective therapeutic effects on cancerous tissue in the interior of the body, scattered cancer cells, or minute initial cancer cells.

Specifically, when an examinee is subjected to energy exposure within not less than 1 min. and not more than 48 hr. (preferably, not less than 30 min. and not more than 36 hr.) after the start of dosing of the magnetic vesicular particle-containing preparation of this invention into the vein of the examinee, the temperature of cancerous tissue near the magnetic vesicular particles is raised and a therapeutic treatment is performed without adverse effect to normal cells. The preparation may also be directly injected near tumorous tissue of the examinee. Exposure of an examinee to energy within not less than 0.5 min. and not more than 36 hr. (preferably, not less than 10 min. and not more than 24 hr.) after the start of injection raises the temperature of tumorous tissue near magnetic vesicular particles. Preferred examples of energy exposure include exposure to an alternating magnetic field and exposure to ultrasonic waves. The exposure to an alternating magnetic field is conducted preferably at a frequency of 25 to 500 kHz.

Preferred embodiments of a diagnostic therapeutic system using the magnetic vesicular particle-containing preparation of this invention will be now be described with reference to a drawing. The embodiment shown in the drawing is one of the embodiments of this invention, but the invention is by no means limited to this.

As shown in FIG. 3, diagnostic therapeutic system 10 relating to this invention comprises:

automatic injection device 20 for automatically giving a preparation containing magnetic vesicular particles of this invention to an examinee,
diagnostic device 30 provided with first exposure section 32 for subjecting the preparation-given examinee to exposure to an ultrasonic wave, an electromagnetic wave or an X-ray, and imaging section 34 for scanning a tumor site in which the magnetic vesicular particles are accumulated under the exposure to an ultrasonic wave, an electromagnetic wave or an X-ray,
therapeutic device 40 provided with second exposure section 42 of subjecting the tumor site in which the magnetic vesicular particles are accumulated to exposure to an alternating magnetic field or an ultrasonic wave, and temperature measuring section 44 for measuring a temperature of the tumor site and that of a normal site near the tumor site under the exposure to an alternating magnetic field or an ultrasonic, and
control device 50 which is connected to the automatic injection device 20, the diagnostic apparatus 30 and the therapeutic apparatus 40 through network 60 and which controls an operation of each of these apparatuses and conducts control among these apparatuses.

As the automatic injection device 20 are usable conventionally known ones. A radiographic imaging diagnostic device, a nuclear magnetic resonance imaging diagnostic device or an ultrasonic imaging diagnostic device is usable as the diagnostic device 30. A focused ultrasonic heating device or an alternating magnetic field heating device is usable as the therapeutic device 40. Preferably, the automatic injection device 20, the diagnostic device 30 and the therapeutic device 40 are integrated so as to concurrently perform diagnosis and therapy.

Any apparatus which is provided with a control section (CPU), an operating section such as a keyboard and a mouse and a display section such as a memory and a display, is applicable as the foregoing control device 50, and examples thereof includes a computer. The network 60 may be connected through information communication network, such as an internet, LAN (Local Area Network) or WAN (Wide Area Network). Connection of terminals of these is unconcerned with being with wire or wireless.

The diagnostic therapeutic system 10 preferably has a patient information database. The control device 50 can access the database according to its need to obtain information of an examinee, whereby an approximate position of the tumor site and constitution or health conditions of the examinee can be confirmed in advance.

In the diagnostic therapeutic system 10, first, the automatic injection device 20 automatically gives a preparation containing magnetic vesicular particles to a patient whose tumor site is to be examined and treated. Specifically, when a patient is taken in the diagnostic therapeutic system, first of all, information for specifying the patient is obtained from an IC tag or a biometric certification such as fingerprints or an iris of the patient. The control device 50 accesses the database based on such information to specify the patient. Then, the control device determines the kind or dose of the preparation containing magnetic vesicular particles from the data (chart) of the patient and transmits the automatic injection device 20. The automatic injection device 20 receives the data such as kind or dose from the control device 50 and then doses a preparation containing magnetic vesicular particles to the patient according to predetermined items.

When completing the dose of the preparation, the automatic injection device 20 transmits a signal of completion of the dose to the control device 50. Subsequently, the control device 50 transmits a signal of starting diagnosis of the tumor site to the diagnostic device 30.

The diagnostic device 30 having received the signal allows the first exposure section 32 to expose the patient to ultrasonic, electromagnetic wave or X-ray and further allows the imaging section 34 to scan the tumor site in which magnetic vesicular particles are accumulated. The image data obtained by scanning in the imaging section 34 is transmitted the control device 50. The control device 50 confirms contrast of the image based on the previously inputted data to specify the tumor site in which magnetic vesicular particles are accumulated. Preferably, the system 10 is provided with a display section such as a monitor to artificially confirm the image scanned in the imaging section 34.

The control device 50, which has specified the tumor site, allows the data regarding the position of the tumor site to be contained in the database of the patient and further transmits the information regarding the position of the tumor site to the therapeutic device 40.

The therapeutic device 40 which has received the data regarding the position of the tumor site, allows the second exposure section 42 to expose the patient to an alternating magnetic field or an ultrasonic and further allows the temperature measuring section 44 to measure the temperature of the tumor site and the vicinity thereof. In the temperature measurement of the temperature measuring section 44, the temperature in vivo is non-invasively measured without embedding a sensor, applying temperature variation being represented as variation of resonance frequency. Preferably, the temperature measuring section 44 conducts a temperature measurement which calculates the temperature from a vertical relaxation time by a signal intensity method, a proton chemical shift by a phase method or a diffusion coefficient by a diffusion image method, each of which uses a nuclear magnetic resonance imaging apparatus, or from values obtained in microwave radiometry using plural frequencies.

When the control device 50 judges that the tumor site is too small to perform an effective treatment or judges that a treatment is to be conducted with confirming the tumor site, the control device 50 controls the first exposure section 32 to allow the tumor site to be exposed to an ultrasonic wave, electromagnetic wave or X-ray and the second exposure section 42 to allow the tumor site to be exposed to an alternating magnetic field or ultrasonic to perform therapy, while scanning, by the imaging section, the tumor site in which magnetic vesicular particles are accumulated.

The temperature measuring section 44, which has measured the temperature of the tumor site and a normal site near the tumor site, successively sends the results thereof to the control device 50. The control device 50, which has received the measurement results and confirmed that the tumor site has been raised to a prescribed temperature (e.g., 42 °C), sends the second exposure section 42 an order of stopping the exposure to an alternating magnetic field or an ultrasonic wave. After reaching the prescribed temperature, the control device may control the second exposure section 42 so as to continue exposure to an alternating magnetic field or an ultrasonic for a given period of time. In cases when the tumor site has not reached a prescribed temperature and the normal site near the tumor site has reached the prescribed temperature, the control device 50 sends the second exposure section 42 an order of stopping the exposure to an alternating magnetic field or an ultrasonic.

As described above, the diagnostic therapeutic system can perform confirmation of a tumor site to the therapy thereof in a single system. Thus, diagnosis and therapy, which were conventionally conducted separately, are concurrently performed and lightens the burden on a patient, performing diagnosis and therapy effectively and efficiently.

There has been described the diagnostic therapeutic system of this invention but the invention is not limited thereto, and various changes and modifications can be made therein without departing from the object of this invention.

## EXAMPLES

The present invention will be further described with reference to specific examples but the invention is by no means limited to these.

Mercapto-group containing Compound 4

Using lithium chloride, a hydroxy group of hexamethylene glycol monomethyl ether was converted to chlorine according to the conventional method and converted chlorine was further converted to a mercapto group to obtain a mercapto group-containing organic compound (A-1). Similarly, mercapto group-containing organic compounds (A-2,

A-3) were obtained.

A-1 : $CH_3(OCH_2CH_2)_6SH$
A-2: $CH_3(OCH_2CH_2)SH$
A-3: $CH_3(CH_2)_{17}SH$

Organic compounds which are to be added in the formation of magnetic microparticles are as follows.

B-1: $HOCH_2CH_2SH$
B-2: cysteine
B-3: dithiothreitol
C: stearic acid chloride

Example 1

Formation Method 1 of Magnetic Microparticle

To 50 ml of an aqueous 1 wt% PVA solution were added magnetic microparticles comprised of ferrite, as shown in Table 1, 8.5 mg of $HAuCl_4$ (4.9 mg Au and its concentration of 0.5 mmol/L), and 0.472 ml of 2-propanol to prepare dispersion 1 of raw materials. This dispersion 1 was enclosed in a glass vial (volume of 70 ml) and was exposed to γ-ray at a dose 3 kGy/h (radiation source: cobalt 60 radiation source, energy of γ-ray photon: 1.25 MeV) and a radiation source power of 7,000 curie) over a period of time shown in Table 1 at room temperature, while stirring.

Subsequently, a magnetic field was applied to the thus exposed dispersion from the outside of the glass vial, using a columnar magnet of 30 mm in diameter and 10 mm in height (at a magnetic flux density of 445 mT) and allowed to stand for 12 hrs. Thereafter, the vial was opened and the supernatant was recovered and theresidue was washed with water, ethanol and methyl ethyl ketone and dried to obtain powdery magnetic microparticles A having particulate gold attached.

The thus obtained magnetic microparticles A was dispersed in water again. Further thereto, a mercapto group containing organic compound, as shown in Table 1, was added so as to have an initial concentration of 100 μmol/L. After stirred for 2 hrs., magnetic microparticles were magnetically collected, separated by using a filter, washed with water, ethanol and methyl ethyl ketone, and dried. Magnetic microparticle B was thus obtained in which particulate gold was attached to the magnetic particle surface and a mercapto group-containing organic compound was bonded to the particulate gold. The particle size (r1) of magnetic microparticles having particulate gold attached and the particle size (r2) of the particulate gold were each determined as an average value for 20 particles observed by a transmission electron microscope.

Attachment of particulate gold to the surface of magnetic microparticles was confirmed by determining absorbance of a solution in which magnetic microparticles (A) were dispersed and observing surface plasmon absorption due to particulate gold. Bonding of a mercapto group-containing organic compound to particulate gold was confirmed in such a manner that using N-(9-acrydinyl)maleimide as a fluorescent labeling agent for a mercapto group, dissolved in dioxane, a solution immediately after adding a mercapto group-containing compound and a solution after stirring were each taken out in an equivalent amount and diluted in the same dilution ratio, and fluorescence absorption of supernatant was higher in immediately after addition.

Example 2

Formation Method 2 of Magnetic Microparticle

Powdery magnetic microparticles A having particulate gold attached thereto were prepared similarly to the foregoing method and introduced into hexane, and dispersed using an ultrasonic dispersing machine. To this hexane dispersion of magnetic microparticles was added an acid chloride (organic compound C: stearic acid chloride) in an amount so as to have an initial concentration of 100 $\mu$mol/L to allow organic compound C to react with the organic compound B chemically bonded to particulate gold. Subsequently, isopropyl alcohol was added to esterify excessive organic compound C. Then, magnetic microparticles were separated by magnetic separation and washed with acetone and distilled water to obtain magnetic microparticles C to which organic compound B is bonded via particulate gold, in which organic compound C is chemically bonded to organic compound B. The particle size (r1) of magnetic microparticles having particulate gold attached and the particle size (r2) of the particulate gold were each determined as an average value for 20 particles observed by a transmission electron microscope.
Attachment of particulate gold to magnetic microparticles and bonding of an organic compound to the particulate gold were confirmed similarly to the foregoing formation method 1.

Example 3

Formation Method 3 of Magnetic Microparticle

To 50 ml of an aqueous 1 wt% PVA solution were added magnetic microparticles comprised of ferrite, as shown in Table 1, 8.5 mg of $HAuCl_4$ (4.9 mg Au and its concentration of 0.5 mmol/L), and 0.472 ml of 2-propanol to prepare dispersion 1 of raw materials. This dispersion 1 was enclosed in a glass vial (volume of 70 ml) and was exposed to ultrasonic (at a frequency of 200 kHz and a power of 200 W over a period of 20 min.). Subsequently, a magnetic field was applied to the thus exposed dispersion from the outside of the glass vial, using a columnar magnet of 30 mm in diameter and 10 mm in height (at a magnetic flux density of 445 mT) and allowed to stand for 1-24 hrs. Thereafter, the vial was opened and the supernatant was recovered and the residue was washed with water, ethanol and methyl ethyl ketone and dried to obtain powdery magnetic microparticles D having particulate gold attached.
The thus obtained magnetic microparticles D was dispersed in water again. Further thereto, glutathione was added so as to have an initial concentration of 100 $\mu$mol/L. After stirred for 2 hrs., magnetic microparticles were magnetically collected, separated by using a filter, washed with water, ethanol and methyl ethyl ketone, and dried. Magnetic microparticle E was thus obtained in which particulate gold was attached to the magnetic particle surface and a mercapto group-containing organic compound was bonded to the particulate gold. The particle size (r1) of magnetic microparticles having particulate gold attached and the particle size (r2) of particulate gold were each determined as an average value for 20 particles observed by a transmission electron microscope.
Attachment of particulate gold to magnetic microparticles and bonding of an organic compound to the particulate gold were confirmed similarly to the foregoing formation method 1.

Example 4

Formation Method 4 of Magnetic Microparticle

Powdery magnetic microparticles D having particulate gold attached thereto were prepared similarly to the foregoing method and introduced into hexane, and dispersed using an ultrasonic dispersing machine. To this hexane dispersion of magnetic microparticles was added an acid chloride (organic compound C) in an amount so as to have an initial concentration of 100 $\mu$mol/L to allow organic compound C to react with the organic compound B chemically bonded to particulate gold. Subsequently, isopropyl alcohol was added to esterify excessive organic compound C. Then, magnetic microparticles were separated by magnetic separation and washed with acetone and distilled water to obtain magnetic microparticles F to which organic compound B is bonded via particulate gold in which organic compound C is chemically bonded to organic compound B. The particle size (r1) of magnetic microparticles having particulate gold attached and the particle size (r2) of particulate gold were each determined as an average value for 20 particles observed by a transmission electron microscope.
Attachment of particulate gold to magnetic microparticles and bonding of an organic compound to the particulate gold were confirmed similarly to the foregoing formation method 1.

For comparison, there were prepared magnetic microparticles (magnetic microparticles 26 and 27), not having particulate gold and a mercapto group-containing organic compound, and magnetic microparticles (magnetic microparticles 28 and 29), having particulate gold but not having a mercapto group-containing organic compound, as shown in Table 1.

Magnetic Vesicular Particle

Using magnetic microparticles, as shown in Table 2, magnetic vesicular particles No. 1 to 50 which were covered with lipid membrane, were manufactured according to the following methods.

Example 5

Manufacturing Method 1 of Vesicular Particles

Using magnetic microparticles, as shown in Table 2, magnetic vesicular particles were prepared in the following manner (denoted as manufacturing method 1). In a 10 ml eggplant type flask, 1.5 mg of dipalmitoylphosphatidylcholine (DPPC), 8.5 mg of phosphatidylethanolamine and 20 mg of phosphatidylcholine were dissolved in 2.0 ml of chloroform to form a homogeneous solution. Thereafter, solvents in the solution were removed under reduced pressure and dried overnight in a desiccator to form a DPPC film in the flask.

Subsequently, distilled water was added to the magnetic microparticles described in Table 2 to form a slurry suspension containing the magnetic microparticles at a concentration of 10 $\mu g/\mu l$. The slurry suspension was added into the flask, at 0.5-6.0 $\mu m$ and further thereto was added 0.4 ml of a phosphoric acid-buffered physiological saline of a 10-fold concentration was added. The thus formed mixture was stirred by repeating ultrasonic stirring over 60 sec. and then pausing over 30sec. for a period of 60 min. After completion of ultrasonic stirring, the mixture was subjected to centrifugal separation for 15 min. using a centrifugal separator at a rotation speed of 3,300 rpm. The obtained supernatant was further subjected to centrifugation at a rotation speed of 7,500 rpm for 50 min., whereby lipid membrane-covered magnetic vesicular particles (hereinafter, also denoted simply as magnetic vesicular particles) having a grain size (R) shown in Table 2 were obtained as precipitates.

The grain size of the magnetic vesicular particles was observed using a transmission electron microscope. The grain size (R) is an averaged value of 20 magnetic vesicular particles.

Example 6

Manufacturing Method 2 of Vesicular Particles

In a 10 ml eggplant type flask, dimethyloctadecylammonium bromide, dioleylphosphatidylcholine (DOPE) and C-2 ceramide at a ratio of 1:1:2 (in a total amount of 2 ml) were dissolved in 2.0 ml of chloroform to form a homogeneous solution. Thereafter, solvents of the solution were removed under reduced pressure and dried overnight in a desiccator to form a DPPC film in the flask.

Subsequently, distilled water was added to the magnetic microparticles described in Table 2 to form a slurry suspension containing magnetic microparticles at a concentration of 10 $\mu g/\mu l$. The slurry suspension was added into the flask, within an amount of 0.5 to 6.0 $\mu m$ and further thereto, a phosphoric acid-buffered physiological saline at a 10-fold concentration was added in an amount of 1/10 of the volume of the slurry suspension of magnetic microparticles. The thus formed mixture was stirred by repeating ultrasonic stirring over 60 sec. and pausing over 30sec. for a period of 60 min. After completion of ultrasonic stirring, the mixture was subjected to centrifugal separation for 15 min. using a centrifugal separator at a rotation speed of 3,300 rpm. The obtained supernatant was further subjected to centrifugation at a rotation speed of 7,500 rpm for 50 min., whereby phospholipid membrane-covered magnetic vesicular particles having a grain size (R) shown in Table 2 were obtained as precipitates.

The grain size (R) of the phospholipid membrane-covered magnetic vesicular particles was determined similarly to the foregoing manufacturing method of the lipid membrane-covered magnetic vesicular particles.

Example 7

Manufacturing Method 3 of Vesicular Particles

A mixture of 40 mg of DPPC, 1.2 mg of a block copolymer of polyethyleneoxide and polypropyleneoxide (pluronic F-88, produced by ADEKA Co., Ltd.) and 900 mg of ethanol was placed into a stainless steel autoclave and the interior of the autoclave was heated to 60 °C, then, 13 g of liquid carbon dioxide was added thereto. The pressure within the autoclave was increased from 50 kg/cm$^2$ to 200 kg/cm$^2$ and DPPC was dissolved in the supercritical carbon dioxide, while stirring within the autoclave. Subsequently, physiological saline was added to the magnetic microparticles described in Table 2 to form a slurry suspension containing magnetic microparticles at a concentration of 10 $\mu$g/$\mu$l.

While stirring the supercritical carbon dioxide solution, the foregoing slurry suspension was continuously added thereto within a range of 0.5 to 6.0 ml. The interior of the autoclave was then evacuated to discharge the carbon dioxide. The mixture was subjected to centrifugal separation for 15 min. using a centrifugal separator at a rotation speed of 3,300 rpm. The obtained supernatant was further subjected to centrifugation at a rotation speed of 7,500 rpm for 50 min., whereby phospholipid membrane-covered magnetic vesicular particles having a grain size (R) shown in Table 2 were obtained as precipitates.

The grain size (R) of the phospholipid membrane-covered magnetic vesicular particles was determined similarly to the foregoing manufacturing method of the lipid membrane-covered magnetic vesicular particles.

Example 8

Manufacturing Method 4

Dimethyloctadecylammonium bromide, dioleylphosphatidylcholine (DOPE) and C-2 ceramide were mixed at a ratio of 1:1:2 (in a total amount of 2 ml). The obtained mixture and 900 mg of ethanol was placed into a stainless steel autoclave and the interior of the autoclave was heated to 60 °C, then, 13 g of liquid carbon dioxide was added thereto. The pressure within the autoclave was increased from 50 kg/cm$^2$ to 200 kg/cm$^2$ and DOPE was dissolved in the supercritical carbon dioxide, while stirring within the autoclave. Subsequently, physiological saline was added to the magnetic microparticles described in Table 2 to form a slurry suspension containing magnetic microparticles at a concentration of 10 $\mu$g/$\mu$l.

While stirring supercritical carbon dioxide solution, the foregoing slurry suspension was continuously added thereto within the range of 0.5 to 6.0 ml. The interior of the autoclave was evacuated to discharge carbon dioxide. The mixture was subjected to centrifugal separation for 15 min. using a centrifugal separator at a rotation speed of 3,300 rpm. The obtained supernatant was further subjected to centrifugation at a rotation speed of 7,500 rpm for 50 min., whereby phospholipid membrane-covered magnetic vesicular particles having a grain size (R) shown in Table 2 were obtained as precipitates.

The grain size (R) of the phospholipid membrane-covered magnetic vesicular particles was determined similarly to the foregoing manufacturing method of the lipid membrane-covered magnetic vesicular particles.

Example 9

Manufacturing Method 5

Lipid having a maleimide group (EMC-DPPE) was prepared in accordance with Example 1 of JP-A No. 11-106391. Thus, 1.5 mg of the EMC-DPPE, 8.5 mg of phosphatidylethanolamine, 20 mg of phosphatidylcholine and 900 ml of ethanol were placed into a stainless steel autoclave and the interior of the autoclave was heated to 60 °C, then, 13 g of liquid carbon dioxide was added thereto. The pressure in the interior of the autoclave was increased from 50 kg/cm$^2$ to 200 kg/cm$^2$ and lipid was dissolved in the supercritical carbon dioxide, while stirring within the autoclave. Subsequently, physiological saline was added to the magnetic microparticles described in Table 2 to form a slurry suspension containing magnetic microparticles at a concentration of 10 $\mu$g/$\mu$l.

While stirring the supercritical carbon dioxide solution, the foregoing slurry suspension was continuously added thereto at 0.5-6.0 ml. The interior of the autoclave was evacuated to discharge the carbon dioxide.

To this solution, monoclonal antibody G-22 was attached in accordance with the method described in Example 1 of JP-A No. 11-106391. The mixture was subjected to centrifugal separation for 15 min. using a centrifugal separator at a rotation speed of 3,300 rpm. The obtained supernatant was further subjected to centrifugation at a rotation speed of 7,500 rpm for 50 min., whereby phospholipid membrane-covered magnetic vesicular particles having a grain size (R) shown in Table 2 were obtained as precipitates.

The grain size (R) of the phospholipid membrane-covered magnetic vesicular particles was determined similarly to the foregoing manufacturing method of the lipid membrane-covered magnetic vesicular particles.

Diagnostic and Therapeutic Test

Using the lipid membrane-covered magnetic vesicular particles obtained above, the following tests (Tests 1-6) were conducted.

Test 1

The magnetic vesicular particles shown in Table 4 were each diluted with an isotonic glucose solution to a concentration of 10 mg iron/ml. The thus diluted solution was injected into a vein of rabbits to which experimental tumor VX-2 was transplanted to their liver in different sizes. After the elapse of time shown in Table 4, the rabbits were observed in an ultrasonic imaging diagnostic apparatus to judge the size of discriminable liver cancer. Results thereof are shown in Table 3.

As can be seen from Table 4, it was proved that lipid membrane-covered magnetic vesicular particles of this invention had no problem with respect to discriminable tumor size, as compared to comparative examples and variation in size of discriminable tumor was less than in the comparative samples, even after an elapse of time.

Test 2

The magnetic vesicular particles shown in Table 5 were each diluted with an isotonic glucose solution to a concentration of 10 mg iron/ml. This solution was locally injected into the breast cancer tumor site of mousses into which a cell line of human breast cancer was hypodermically transplanted. After the elapse of time as shown in Table 4, the rabbits were observed in a nuclear magnetic resonance imaging diagnostic apparatus to determine the size of discriminable tumors. Results thereof are shown in Table 5.

As can be seen from Table 4, it was proved that lipid membrane-covered magnetic vesicular particles of this invention were discriminable even after an elapse of time, as compared to comparative examples.

Test 3

The magnetic vesicular particles shown in Table 6 were each diluted with an isotonic glucose solution to a concentration of 10 mg iron/ml. This solution was injected into a vein of rats into which a cell line of human malignant glioma had been transplanted. After an elapse of time as shown in Table 5, the rats were observed in a nuclear magnetic resonance imaging diagnostic apparatus to determine the size of discriminable tumors. Results thereof are shown in Table 6.

As can be seen from Table 6, it was proved that lipid membrane-covered magnetic vesicular particles of this invention were discriminable even after an elapse of time, as compared to comparative examples.

Test 4

The magnetic vesicular particles shown in Table 7 were each diluted with an isotonic glucose solution to a concentration of 10 mg iron/ml. This solution was locally injected into the breast cancer site of a mouse into which a cell line of low molecular type line cancer, originated from human lung was transplanted immediately below the pleura. After 30 min. or 24 hr., exposure to an alternating magnetic field was conducted under the following conditions:

  frequency: 375 Hz,
  coil to generate a magnetic field: 300 mm in diameter, magnetic field intensity: 6 mT,
  current: 225 A,
  output: 3 KW, and
  distance from an applicator: 20 mm.

A fiberoptic thermometer was arranged in and near the tumor site to determine temperatures of tumor and normal sites. The time of the tumor site reaching 43 °C was determined, at which time the temperature of the normal site was also determined. Results thereof are shown in Table 6.

As can be seen from Table 7, it was proved that when thermotherapy was conducted, the lipid membrane-covered magnetic vesicular particles of this invention efficiently raised the temperature of only the tumor site within a short period of time, enabling performance 0f efficient thermotherapy irrespective of elapse of time after local injection, as compared to comparative examples.

Test 5

The magnetic vesicular particles shown in Table 8 were each diluted with an isotonic glucose solution to a concentration of 10 mg iron/ml. This solution was injected into veins of rats into which a cell line of human malignant glioma was transplanted. After 1 hr. or the time shown in Table 7, exposure to an alternating magnetic field was continuously conducted under the following conditions:

frequency: 370 Hz,
applicator to generate a magnetic field: 310 mm in coil diameter,
magnetic field intensity: 8 mT, current: 300 A,
output: 5 KW, and
distance from the applicator: 20 mm.

A fiberoptic thermometer was arranged in the tumor site to determine temperatures of the tumor and the normal site. The time of the tumor site reaching 43 °C was determined, at which time the temperature of the normal site was also determined. Results thereof are shown in Table 8.

As can be seen from Table 8, it was proved that when thermotherapy was conducted, the lipid membrane-covered magnetic vesicular particles of this invention efficiently raised a temperature of only a tumor site within a short period of time, as compared to the comparative examples. It was further shown that enhanced accumulation at the tumor site enables performance of efficient therapy without newly supplying magnetic grains when applying therapy a few times. Test 6

The magnetic vesicular particles shown in Table 9 were each diluted with an isotonic glucose solution to a concentration of 10 mg iron/ml. This solution was injected through intravenous injection to mousses into which a cell line of human breast cancer was hypodermically transplanted. After 2 hr. and 24 hr., exposure to an alternating magnetic field was conducted similarly to the foregoing test 5. After 6 days since the first intravenous injection, a second intravenous injection was conducted, and again after 2 hr. and 24 hr., exposure to an alternating magnetic field was conducted similarly to the foregoing test 5. Again, after 15 days since the second intravenous injection, a third intravenous injection was conducted.

After 1 hr. since the respective first, second and third injections of the magnetic vesicular particles, observation was conducted in a nuclear magnetic resonance imaging diagnostic apparatus to determine the size of a glioma tumor site from a diagnosis image. Results thereof are shown in Table 8. In any case of the foregoing, capability of achieving contrast as a contrast medium for tumor was at a level acceptable in practice.

As can be seen from Table 9, it was proved that magnetic vesicular particles of this invention can not only provide both functions as an imaging agent (or contrast medium) and a therapeutic agent but also efficiently function as a therapeutic agent.

Table 1

| Magnetic Microparticle No. | *1 | Ferrite Particle | | Organic Compound A | Organic Compound B | *3 | Particle Size | |
|---|---|---|---|---|---|---|---|---|
| | | *2 | Amount (mg) | | | | r1 (nm) | r2 (nm) |
| 1 | 1 | 20 | 50 | A-1 | | 3 | 20 | 5 |
| 2 | 1 | 20 | 50 | A-2 | | 3 | 20 | 5 |
| 3 | 1 | 20 | 50 | A-3 | | 3 | 20 | 5 |
| 4 | 2 | 20 | 50 | | B-1 | 3 | 20 | 5 |
| 5 | 2 | 20 | 50 | | B-2 | 3 | 20 | 5 |
| 6 | 2 | 20 | 50 | | B-3 | 3 | 20 | 5 |
| 7 | 3 | 20 | 50 | A-2 | | 2 | 20 | 4 |
| 8 | 3 | 20 | 50 | A-3 | | 2 | 20 | 4 |
| 9 | 4 | 20 | 50 | | B-1 | 2 | 20 | 4 |
| 10 | 4 | 20 | 50 | | B-3 | 2 | 20 | 4 |
| 11 | 1 | 5 | 50 | A-2 | | 3 | 5 | 4 |
| 12 | 1 | 10 | 50 | A-2 | | 3 | 10 | 4 |
| 13 | 1 | 15 | 50 | A-2 | | 3 | 15 | 5 |
| 14 | 1 | 25 | 50 | A-2 | | 3 | 25 | 5 |
| 15 | 1 | 30 | 50 | A-2 | | 3 | 30 | 5 |
| 16 | 1 | 20 | 40 | A-2 | | 3 | 20 | 5 |

(continued)

| Magnetic Microparticle No. | *1 | Ferrite Particle | | Organic Compound A | Organic Compound B | *3 | Particle Size | |
|---|---|---|---|---|---|---|---|---|
| | | *2 | Amount (mg) | | | | r1 (nm) | r2 (nm) |
| 17 | 1 | 20 | 30 | A-2 | | 3 | 20 | 5 |
| 18 | 1 | 20 | 20 | A-2 | | 3 | 20 | 4 |
| 19 | 1 | 20 | 10 | A-2 | | 3 | 20 | 4 |
| 20 | 1 | 20 | 50 | A-2 | | 1 | 20 | 3 |
| 21 | 1 | 20 | 50 | A-2 | | 6 | 20 | 7 |
| 22 | 1 | 20 | 50 | A-2 | | 9 | 20 | 8 |
| 23 | 1 | 20 | 50 | A-2 | | 12 | 20 | 9 |
| 24 | 2 | 20 | 50 | | | 6 | 20 | 7 |
| 25 | 2 | 20 | 50 | | | 6 | 20 | 7 |
| 26 | - | 20 | 50 | - | - | - | 20 | 0 |
| 27 | - | 30 | 50 | - | - | - | 20 | 0 |
| 28 | 1 | 20 | 50 | - | - | 3 | 20 | 5 |
| 29 | 1 | 30 | 50 | - | - | 3 | 30 | 5 |

*1: Formation method No. of magnetic microparticles
*2: Particle Size (nm)
*3: γ-ray Exposure Time (hr)

Table 2

| Magnetic Vesicular Particle No. | Manufacturing Method | Magnetic Microparticle No. | r (nm)*1 | R (nm)*2 | R/ (rx100)*3 | Remark |
|---|---|---|---|---|---|---|
| 1 | 1 | 1 | 20 | 100 | 0.050 | Inv. |
| 2 | 1 | 2 | 20 | 100 | 0.050 | Inv. |
| 3 | 1 | 3 | 20 | 100 | 0.050 | Inv. |
| 4 | 1 | 4 | 20 | 100 | 0.050 | Inv. |
| 5 | 1 | 5 | 20 | 100 | 0.050 | Inv. |
| 6 | 1 | 6 | 20 | 100 | 0.050 | Inv. |
| 7 | 1 | 7 | 20 | 100 | 0.050 | Inv. |
| 8 | 1 | 8 | 20 | 100 | 0.050 | Inv. |
| 9 | 1 | 9 | 20 | 100 | 0.050 | Inv. |
| 10 | 1 | 10 | 20 | 100 | 0.050 | Inv. |
| 11 | 1 | 26 | 20 | 100 | 0.050 | Comp. |
| 12 | 1 | 28 | 20 | 100 | 0.050 | Comp. |
| 13 | 2 | 11 | 5 | 100 | 0.200 | Inv. |
| 14 | 2 | 11 | 5 | 150 | 0.300 | Inv. |
| 15 | 2 | 11 | 5 | 180 | 0.360 | Comp. |
| 16 | 2 | 12 | 10 | 100 | 0.100 | Inv. |

(continued)

| Magnetic Vesicular Particle No. | Manufacturing Method | Magnetic Microparticle No. | r (nm)*1 | R (nm)*2 | R/ (rx100)*3 | Remark |
|---|---|---|---|---|---|---|
| 17 | 2 | 13 | 15 | 100 | 0.067 | Inv. |
| 18 | 2 | 2 | 20 | 100 | 0.050 | Inv. |
| 19 | 2 | 14 | 25 | 100 | 0.040 | Inv. |
| 20 | 2 | 15 | 30 | 40 | 0.013 | Comp. |
| 21 | 2 | 15 | 30 | 100 | 0.033 | Inv. |
| 22 | 2 | 15 | 30 | 150 | 0.050 | Inv. |
| 23 | 2 | 27 | 30 | 100 | 0.033 | Comp. |
| 24 | 2 | 28 | 30 | 100 | 0.033 | Comp. |
| 25 | 3 | 1 | 20 | 100 | 0.050 | Inv. |
| *1: Magnetic microparticle size r (nm) *2: Vesicular particle size R (nm) *3: Size ratio R/ (rx100) | | | | | | |

Table 3

| Magnetic Vesicular Particle No. | Manufacturing Method | Magnetic Microparticle r No. | (nm) *1 | R (nm) *2 | R/ (rx100)*3 | Remark |
|---|---|---|---|---|---|---|
| 26 | 3 | 2 | 20 | 100 | 0.050 | Inv. |
| 27 | 3 | 3 | 20 | 100 | 0.050 | Inv. |
| 28 | 3 | 26 | 20 | 100 | 0.050 | Comp. |
| 29 | 3 | 28 | 20 | 100 | 0.050 | Comp. |
| 30 | 4 | 16 | 20 | 100 | 0.050 | Inv. |
| 31 | 4 | 17 | 20 | 100 | 0.050 | Inv. |
| 32 | 4 | 18 | 20 | 100 | 0.050 | Inv. |
| 33 | 4 | 19 | 20 | 100 | 0.050 | Inv. |
| 34 | 4 | 20 | 20 | 100 | 0.050 | Inv. |
| 35 | 4 | 2 | 20 | 100 | 0.050 | Inv. |
| 36 | 4 | 21 | 20 | 100 | 0.050 | Inv. |
| 37 | 4 | 22 | 20 | 100 | 0.050 | Inv. |
| 38 | 4 | 23 | 20 | 100 | 0.050 | Inv. |
| 39 | 4 | 24 | 20 | 100 | 0.050 | Inv. |
| 40 | 4 | 25 | 20 | 100 | 0.050 | Inv. |
| 41 | 4 | 26 | 20 | 100 | 0.050 | Comp. |
| 42 | 4 | 28 | 20 | 100 | 0.050 | Comp. |
| 43 | 5 | 1 | 20 | 100 | 0.050 | Inv. |
| 44 | 5 | 2 | 20 | 100 | 0.050 | Inv. |
| 45 | 5 | 3 | 20 | 100 | 0.050 | Inv. |

(continued)

| Magnetic Vesicular Particle No. | Manufacturing Method | Magnetic Microparticle r No. | (nm) *1 | R (nm) *2 | R/ (rx100)*3 | Remark |
|---|---|---|---|---|---|---|
| 46 | 5 | 4 | 20 | 100 | 0.050 | Inv. |
| 47 | 5 | 5 | 20 | 100 | 0.050 | Inv. |
| 48 | 5 | 6 | 20 | 100 | 0.050 | Inv. |
| 49 | 5 | 26 | 20 | 100 | 0.050 | Comp. |
| 50 | 5 | 28 | 20 | 100 | 0.050 | Comp. |

*1: Magnetic microparticle size r (nm)
*2: Vesicular particle size R (nm)
*3: Size ratio R/(r×100)

Table 4

| Magnetic Vesicular Particle No. | Size of Discriminable Tumor (nm) | | | | | Remark |
|---|---|---|---|---|---|---|
| | After 30 min. | After 1 hr. | After 6 hr. | After 12 hr. | After 24 hr. | |
| 1 | 5 | 5 | 8 | 10 | 10 | Inv. |
| 2 | 5 | 5 | 8 | 10 | 10 | Inv. |
| 3 | 5 | 5 | 8 | 10 | 10 | Inv. |
| 4 | 5 | 5 | 8 | 10 | 10 | Inv. |
| 5 | 5 | 5 | 8 | 10 | 10 | Inv. |
| 6 | 5 | 5 | 8 | 10 | 10 | Inv. |
| 7 | 5 | 5 | 8 | 10 | 10 | Inv. |
| 8 | 5 | 5 | 8 | 10 | 10 | Inv. |
| 9 | 5 | 5 | 8 | 10 | 10 | Inv. |
| 10 | 5 | 5 | 8 | 10 | 10 | Inv. |
| 11 | 5 | 8 | 15 | -* | -* | Comp. |
| 12 | 5 | 8 | 15 | -* | -* | Comp. |
| 25 | 5 | 5 | 8 | 10 | 10 | Inv. |
| 26 | 5 | 5 | 8 | 10 | 10 | Inv. |
| 27 | 5 | 5 | 8 | 10 | 10 | Inv. |
| 28 | 5 | 8 | 15 | -* | -* | Comp. |
| 29 | 5 | 8 | 15 | -* | -* | Comp. |
| 31 | 5 | 5 | 5 | 8 | 10 | Inv. |
| 32 | 5 | 5 | 5 | 8 | 10 | Inv. |
| 33 | 5 | 5 | 5 | 8 | 10 | Inv. |
| 34 | 5 | 5 | 5 | 8 | 10 | Inv. |
| 35 | 5 | 5 | 5 | 8 | 10 | Inv. |
| 36 | 5 | 5 | 5 | 8 | 10 | Inv. |
| 37 | 5 | 5 | 5 | 8 | 10 | Inv. |
| 38 | 5 | 5 | 5 | 8 | 10 | Inv. |

(continued)

| Magnetic Vesicular Particle No. | Size of Discriminable Tumor (nm) | | | | | Remark |
|---|---|---|---|---|---|---|
| | After 30 min. | After 1 hr. | After 6 hr. | After 12 hr. | After 24 hr. | |
| 39 | 5 | 5 | 5 | 8 | 10 | Inv. |
| 40 | 5 | 5 | 5 | 8 | 10 | Inv. |
| 41 | 5 | 8 | 10 | -* | -* | Comp. |
| 42 | 5 | 8 | 10 | -* | -* | Comp. |
| *: Function as a contrast medium was not noted at all. | | | | | | |

Table 5

| Magnetic Vesicular Particle No. | Size of Discriminable Tumor (nm) | | | | | Remark |
|---|---|---|---|---|---|---|
| | After 30 min. | After 1 hr. | After 6 hr. | After 12 hr. | After 24 hr. | |
| 13 | 5 | 5 | 5 | 8 | 10 | Inv. |
| 14 | 5 | 5 | 5 | 8 | 10 | Inv. |
| 15 | 8 | 8 | 8 | 10 | 15 | Comp. |
| 16 | 5 | 5 | 5 | 8 | 10 | Inv. |
| 17 | 5 | 5 | 5 | 8 | 10 | Inv. |
| 18 | 5 | 5 | 5 | 8 | 10 | Inv. |
| 19 | 5 | 5 | 5 | 8 | 10 | Inv. |
| 20 | 5 | 8 | 10 | 15 | 15 | Comp. |
| 21 | 5 | 5 | 5 | 8 | 10 | Inv. |
| 22 | 5 | 5 | 5 | 8 | 10 | Inv. |
| 23 | 5 | 8 | 15 | -* | -* | Comp. |
| 24 | 5 | 8 | 15 | -* | -* | Comp. |
| 43 | 5 | 5 | 5 | 5 | 8 | Inv. |
| 44 | 5 | 5 | 5 | 5 | 8 | Inv. |
| 45 | 5 | 5 | 5 | 5 | 8 | Inv. |
| 46 | 5 | 5 | 5 | 5 | 8 | Inv. |
| 47 | 5 | 5 | 5 | 5 | 8 | Inv. |
| 48 | 5 | 5 | 5 | 5 | 8 | Inv. |
| 49 | 5 | 8 | 10 | 15 | -* | Comp. |
| 50 | 5 | 8 | 10 | 15 | -* | Comp. |
| *: Function as a contrast medium was not noted at all | | | | | | |

Table 6

| Magnetic Vesicular Particle No. | Size of Discriminable Tumor (nm) | | | | | | Remark |
|---|---|---|---|---|---|---|---|
| | After 30 min. | After 1 hr. | After 6 hr. | After 12 hr. | After 24 hr. | After 36 hr. | |
| 2 | 5 | 5 | 8 | 10 | 15 | 15 | Inv. |
| 30 | 5 | 5 | 5 | 8 | 8 | 10 | Inv. |

(continued)

| Magnetic Vesicular Particle No. | Size of Discriminable Tumor (nm) | | | | | | Remark |
| | After 30 min. | After 1 hr. | After 6 hr. | After 12 hr. | After 24 hr. | After 36 hr. | |
|---|---|---|---|---|---|---|---|
| 31 | 5 | 5 | 5 | 5 | 8 | 10 | Inv. |
| 32 | 5 | 5 | 5 | 5 | 8 | 10 | Inv. |
| 33 | 5 | 5 | 5 | 5 | 8 | 10 | Inv. |
| 34 | 5 | 5 | 5 | 5 | 8 | 10 | Inv. |
| 35 | 5 | 5 | 5 | 5 | 8 | 10 | Inv. |
| 36 | 5 | 5 | 5 | 5 | 8 | 10 | Inv. |
| 37 | 5 | 5 | 8 | 8 | 10 | 15 | Inv. |
| 38 | 5 | 5 | 8 | 8 | 10 | 15 | Inv. |
| 39 | 5 | 5 | 8 | 10 | 10 | 15 | Inv. |
| 40 | 5 | 5 | 8 | 10 | 10 | 15 | Inv. |
| 41 | 5 | 8 | 15 | -*1 | -*1 | -*1 | Comp. |
| 42 | 5 | 8 | 15 | -*1 | -*1 | -*1 | Comp. |
| 43 | 5 | 5 | 5 | 5 | 5 | 8 | Inv. |
| 44 | 5 | 5 | 5 | 5 | 5 | 8 | Inv. |
| 45 | 5 | 5 | 5 | 5 | 5 | 8 | Inv. |
| 46 | 5 | 5 | 5 | 5 | 5 | 8 | Inv. |
| 47 | 5 | 5 | 5 | 5 | 5 | 8 | Inv. |
| 48 | 5 | 5 | 5 | 5 | 5 | 8 | Inv. |
| 49 | 5 | 8 | 10 | 15 | -* | -* | Comp. |
| 50 | 5 | 8 | 10 | 15 | -* | -* | Comp. |
| *: Function as a contrast medium was not noted at all | | | | | | | |

Table 7

| Magnetic Vesicular Particle No. | Normal Site Temperature*1 (min) (°C) | Heating (1)*2 | | Heating (2)*3 | | Remark |
| | | Time *4 (min) | Temparature *5 (°C) | Time*4 (min) | Temparature*5 (°C) | |
|---|---|---|---|---|---|---|
| 2 | 33.3 | 4.5 | 34.3 | 15.0 | 38.1 | Inv. |
| 30 | 33.1 | 4.0 | 34.2 | 7.5 | 35.4 | Inv. |
| 31 | 32.6 | 4.0 | 34.1 | 7.5 | 35.2 | Inv. |
| 32 | 32.8 | 4.0 | 33.9 | 7.0 | 34.8 | Inv. |
| 33 | 33.0 | 4.0 | 34.0 | 7.0 | 35.0 | Inv. |
| 34 | 33.4 | 3.5 | 33.9 | 6.5 | 34.9 | Inv. |
| 35 | 32.5 | 4.0 | 33.9 | 7.0 | 35.2 | Inv. |
| 36 | 33.4 | 4.5 | 34.3 | 8.0 | 35.8 | Inv. |
| 37 | 32.9 | 4.5 | 34.0 | 10.0 | 36.2 | Inv. |
| 38 | 32.8 | 5.0 | 34.4 | 12.0 | 36.9 | Inv. |

(continued)

| Magnetic Vesicular Particle No. | Normal Site Temperature*1 (min) (°C) | Heating (1)*2 | | Heating (2)*3 | | Remark |
|---|---|---|---|---|---|---|
| | | Time *4 (min) | Temparature *5 (°C) | Time*4 (min) | Temparature*5 (°C) | |
| 41 | 33.3 | 4.5 | 34.4 | -*6 | -*6 | Compo. |
| 42 | 32.9 | 4.5 | 34.1 | -*6 | -*6 | Comp. |
| *1: normal site temperature before subjected to alternating magnetic field heating<br>*2: alternating magnetic field heating at 30 min after injection<br>*3: alternating magnetic field heating at 24 hr. after injection<br>*4: time when the temperature reached 43 °C<br>*5: normal site temperature (°C)<br>*6: no temperature increase was noted even after subjected to alternating magnetic field heating for 30 min. | | | | | | |

Table 8

| *1 | Normal Site Temperature*2 (°C) | Heating (1)*3 | | Heating (2)*4 | | Heating (3)*5 | | Remark |
|---|---|---|---|---|---|---|---|---|
| | | Time*6 (min) | Temperature *7 (°C) | Time*6 (min) | Temparature*7 (°C) | Time*6 (min) | Temparature*7 (°C) | |
| 43 | 32.9 | 3.0 | 33.5 | 3.5 | 33.9 | 5.5 | 34.6 | Inv. |
| 2 | 33.5 | 4.5 | 34.6 | 7.0 | 35.1 | 17.0 | 39.1 | Inv. |
| 18 | 33.4 | 4.0 | 34.2 | 4.5 | 34.4 | 7.5 | 35.4 | Inv. |
| 26 | 33.1 | 4.5 | 39.4 | 5.0 | 34.6 | 9.0 | 35.9 | Inv. |
| 35 | 32.8 | 4.0 | 34.1 | 4.5 | 34.5 | 7.5 | 35.5 | Inv. |
| 44 | 33.0 | 3.0 | 33.8 | 3.5 | 34.1 | 5.5 | 34.9 | Inv. |
| 45 | 32.9 | 3.0 | 33.6 | 3.5 | 33.9 | 5.5 | 34.6 | Inv. |
| 46 | 33.0 | 3.0 | 33.7 | 4.0 | 34.2 | 6.0 | 35.1 | Inv. |
| 47 | 33.4 | 3.0 | 34.0 | 4.0 | 34.4 | 6.0 | 35.2 | Inv. |
| 48 | 33.2 | 3.0 | 33.9 | 4.0 | 34.1 | 6.0 | 35.0 | Inv. |
| 49 | 33.1 | 4.5 | 34.6 | 22.0 | 41.1 | -*8 | -*8 | Comp. |
| 50 | 33.2 | 4.5 | 34.8 | 23.5 | 41.6 | -*8 | -*8 | Comp. |

*1: Magnetic Vesicular Particle No.
*2: normal site temperature before subjected to alternating magnetic field heating
*3: alternating magnetic field heating at 1 hr. after injection
*4: alternating magnetic field heating at 6 hr. after injection
*5: alternating magnetic field heating at 24 hr. after injection
*6: time when the temperature reached 43 °C
*7: normal site temperature (°C)
*8: no temperature increase was noted even after subjected to alternating magnetic field heating for 30 min.

Table 9

| Magnetic Vesicular Particle No. | Size of Tumor (mm) | | | Remark |
|---|---|---|---|---|
| | 1st (mm) | 2nd (mm) | 3rd (mm) | |
| 16 | 11 | 8 | -* | Inv. |
| 18 | 12 | 9 | -* | Inv. |
| 21 | 10 | 7 | -* | Inv. |
| 23 | 11 | 9 | 7 | Comp. |
| 43 | 12 | 7 | -* | Inv. |
| 44 | 11 | 6 | -* | Inv. |
| 45 | 10 | 5 | -* | Inv. |
| 50 | 12 | 10 | 8 | Comp. |
| *: No tumor was noted in nuclear magnetic resonance imaging diagnosis. | | | | |

**Claims**

1. A pharmaceutical preparation comprising magnetic vesicular particles, wherein the magnetic vesicular particles each include one or more magnetic microparticles within a lipid membrane, the magnetic microparticle having particulate gold attached to the surface of the magnetic microparticles and further having an organic compound bound to the particulate gold; and the vesicular particles meet the following requirement:

$$0.02 \leq R/(r \times 100) \leq 0.30$$

wherein R represents an average particle size of the magnetic vesicular particles and r represents an average particle size of the magnetic microparticles included in the magnetic vesicular particles.

2. The preparation of claim 1, wherein the vesicular particles meet the following equation:

$$0.03 \leq R/(r \times 100) \leq 0.30$$

3. The preparation of claim 1, wherein the organic compound contains a group selected from the group consisting of a mercapto group, a dithio group, a sulfo group, a thiocarboxyl group, a dithiocarboxyl group, an amino group and a hydroxyl group.

4. The preparation of claim 1, wherein the magnetic microparticles have an average particle size of 5 to 30 nm and are comprised of a ferrite.

5. The preparation of claim 1, wherein the magnetic vesicular particles are liposomes formed of the lipid membrane and including at least one magnetic microparticle within the lipid membrane.

6. The preparation of claim 5, wherein the liposomes exhibit a positive surface charge.

7. The preparation of claim 1, wherein the preparation is used as an imaging agent or a therapeutic agent for tumor.

8. The preparation of claim 7, wherein a physiologically active material or an antitumor-active material is bonded directly or through a linking material to the lipid membrane.

9. The preparation of claim 7, wherein the imaging agent is a contrast medium for use in ultrasonic imaging diagnosis, nuclear magnetic resonance imaging diagnosis or X-ray imaging diagnosis.

10. The preparation of claim 7, wherein, within not less than 1 minute and not more than 48 hours after starting dose of the preparation into a vein of an examinee, a scan is conducted in an ultrasonic imaging diagnosis apparatus, a nuclear magnetic resonance imaging diagnosis apparatus or a radiographic imaging diagnosis apparatus.

11. The preparation of claim 7, wherein, within not less than 0.5 minute and not more than 36 hours after starting injection of the preparation into a region near a tumorous tissue of an examinee, a scan is conducted in an ultrasonic imaging diagnosis apparatus, a nuclear magnetic resonance imaging diagnosis apparatus or a radiographic imaging diagnosis apparatus.

12. The preparation of claim 7, wherein at least one selected from the group consisting of a physiologically functional material, an additively stabilizing material, a medicinally active material, a medicinally active chelating material, an antitumor-active material, an immunopotentiating material, a cell fusion material, and a gene transfer mediating material is bonded directly or through a linking material to an outermost layer of the lipid membrane.

13. The preparation of claim 7, wherein the therapeutic agent is a therapeutic agent for use in thermotherapy.

14. The preparation of claim 13, wherein the thermotherapy is exposure to energy and the preparation is one capable of raising a temperature of a tumorous tissue close to the magnetic grains upon the exposure to energy.

15. The preparation of claim 14, wherein the exposure to energy is exposure to an alternating magnetic field or exposure to an ultrasonic wave.

16. The preparation of claim 14, wherein the exposure to energy is exposure to an alternating magnetic field at a frequency of 25 to 500 Hz.

17. The preparation of claim 14, wherein when an examinee is exposed to an alternating magnetic field or an ultrasonic wave within 1 min. to 48 hr. after the preparation is dosed into a vein of the examinee, the preparation is one capable of raising a temperature of a tumorous tissue close to the magnetic grains.

18. The preparation of claim 14, wherein when an examinee is exposed to an alternating magnetic field or an ultrasonic within 0.5 min. to 36 hr. after the preparation is injected into a portion near a tumorous tissue of the examinee, the preparation is one capable of raising a temperature of the tumorous tissue close to the magnetic vesicular particles.

19. A method of manufacturing a preparation comprising magnetic vesicular particles as claimed in claim 1, the method comprising:

   (a) mixing at least one lipid membrane constituent and a supercritical carbon dioxide to form a mixture (1),
   (b) adding to the mixture (1) a dispersion containing magnetic microparticles having particulate gold attached to the microparticles and further having an organic compound bound to the particulate gold to form a mixture (2), and
   (c) discharging the carbon dioxide from the mixture (2) to form the magnetic vesicular particles including the magnetic microparticles within a lipid membrane.

20. The method of claim 19, wherein in step (b), the magnetic microparticles having particulate gold attached to microparticles are formed by a process comprising (i) dispersing magnetic microparticles in a solution containing gold ions or a gold complex or adding a solution containing gold ions or a gold complex to a dispersion of magnetic microparticles to obtain a mixed solution and (ii) subjecting the mixed solution to exposure to an ultrasonic wave or an ionizing radiation.

21. The method of claim 20, wherein the magnetic microparticles having particulate gold attached to the microparticles and further having an organic compound bound to the particulate gold is formed by a process of dispersing the magnetic microparticles having particulate gold attached to the microparticles into a solution containing the organic compound or adding a solution containing the organic compound to a dispersion containing magnetic microparticles having particulate gold attached to the microparticles.

22. The method of claim 19, wherein the organic compound contains a group selected from the group consisting of a mercapto group, a dithio group, a sulfo group, a thiocarboxyl group, a dithiocarboxyl group, an amino group and a hydroxyl group.

23. A diagnostic therapeutic system for diagnosis and for therapy of a tumor site of an examinee using a preparation comprising magnetic vesicular particles as claimed in claim 1, the system comprising:

an automatic injection device to inject the preparation comprising magnetic vesicular particles into an examinee, a diagnosis device provided with a first exposure section for subjecting the injected examinee to a first exposure to an ultrasonic, an electromagnetic wave or an X-ray and an imaging section for scanning a tumor site in which the magnetic vesicular particles are accumulated upon the first exposure, a therapy device provided with a second exposure section for subjecting the tumor site to a second exposure to an alternating magnetic field or an ultrasonic and a temperature measurement section for measuring a temperature of the tumor site and that of a normal site near the tumor site during the second exposure, and a control device which is connected to each of the automatic injection device, the diagnosis device and the therapy device through a network, for controlling an operation of each of the automatic injection device, the diagnosis device and the therapy device and for performing control among the automatic injection device, the diagnosis device and the therapy device.

24. The system of claim 23, wherein the temperature measurement section performs temperature measurement which is non-invasive for the examinee.

25. The system of claim 24, wherein the temperature measurement section calculates a temperature from a vertical relaxation time by a signal intensity method, a proton chemical shift by a phase method or a diffusion coefficient by a diffusion image method, each of which uses a nuclear magnetic resonance imaging device, or from values obtained in microwave radiometry using plural frequencies.

26. The system of claim 23, wherein the temperature measurement section measures a temperature of a tumor site and that of a normal site near the tumor site and transmits measurement results to the control device, and when the control device confirms from the measurement results that the tumor site has risen to a prescribed temperature, the control device transmits a command to stop the exposure to an alternating magnetic field or an ultrasonic to the second exposure section to control therapy.

27. The system of claim 23, wherein the second exposure section is controlled so that the second exposure section subjects the tumor site to the exposure to an alternating magnetic field or an ultrasonic to perform therapy, while the first exposure section subjects the tumor site to the exposure to an ultrasonic, an electromagnetic wave or an X-ray and the imaging section scans the tumor site to confirm the location thereof, thereby performing therapy with confirming the tumor site.

# FIG. 1

# FIG. 2

# FIG. 3

DIAGNOSTIC THERAPEUTIC SYSTEM

| | |
|---|---|
| AUTOMATIC INJECTION DEVICE | |
| DIAGNOSTIC DEVICE | |
| FIRST EXPOSURE SECTION | |
| IMAGING SECTION | CONTROL DEVICE |
| THERAPEUTIC DEVICE | |
| SECOND EXPOSURE SECTION | |
| TEMPERATURE MEASURING SECTION | |

| | INTERNATIONAL SEARCH REPORT | International application No. |
|---|---|---|
| | | PCT/JP2006/300813 |

**A. CLASSIFICATION OF SUBJECT MATTER**
*A61K41/00*(2006.01), *A61K9/127*(2006.01), *A61K49/00*(2006.01),
*A61P35/00*(2006.01), *A61B5/055*(2006.01), *A61B8/08*(2006.01),
*A61J3/02*(2006.01), *A61N1/40*(2006.01), *A61N2/00*(2006.01)
According to International Patent Classification (IPC) or to both national classification and IPC

**B. FIELDS SEARCHED**

Minimum documentation searched (classification system followed by classification symbols)
A61B5/055, A61B8/08, A61J3/02, A61K9/127, A61K41/00, A61K49/00,
A61N1/40, A61N2/00

Documentation searched other than minimum documentation to the extent that such documents are included in the fields searched
Jitsuyo Shinan Koho        1922-1996   Jitsuyo Shinan Toroku Koho   1996-2006
Kokai Jitsuyo Shinan Koho   1971-2006   Toroku Jitsuyo Shinan Koho   1994-2006

Electronic data base consulted during the international search (name of data base and, where practicable, search terms used)
JMEDPlus(JOIS), JSTPlus(JOIS)

**C. DOCUMENTS CONSIDERED TO BE RELEVANT**

| Category* | Citation of document, with indication, where appropriate, of the relevant passages | Relevant to claim No. |
|---|---|---|
| Y | WO 04/83124 A1 (KANSAI TECHNOLOGY LICENSING ORG CO., LTD., JP), 30 September, 2004 (30.09.04), Claims; example (Family: none) | 1-27 |
| Y | JP 9-110722 A (TORAY IND. INC.), 28 April, 1997 (28.04.97), Full text (Family: none) | 1-27 |
| Y | JP 8-500700 A (BIOQUEST INC.), 23 January, 1996 (23.01.96), Full text & WO 93/26019 A1        & EP 645048 A1 | 1-27 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 15 February, 2006 (15.02.06) | 28 February, 2006 (28.02.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

| | International application No. |
|---|---|
| | PCT/JP2006/300813 |

C (Continuation).   DOCUMENTS CONSIDERED TO BE RELEVANT

| Category* | Citation of document, with indication, where appropriate, of the relevant passages | Relevant to claim No. |
|---|---|---|
| Y | WO 03/22360 A2  (TRITON BIOSYSTEMS INC.),<br>20 March, 2003 (20.03.03),<br>Full text<br>& EP 1409077 A2          & JP 2005-523736 A | 1-27 |
| Y | Akira ITO et al., "Jisei Biryushi o Mochiita<br>Chiryo Gijutsu Kaihatsu", BIO INDUSTRY, 2004,<br>Vol.21, No.8, pages 48 to 54 | 1-27 |
| Y | JP 2002-528472 A  (PHOTOGEN INC.),<br>03 September, 2002 (03.09.02),<br>Full text<br>& WO 00/25665 A1        & EP 1126782 A1<br>& KR 2001080932 A        & CN 1325284 A | 23-27 |

Form PCT/ISA/210 (continuation of second sheet) (April 2005)

**INTERNATIONAL SEARCH REPORT**

| International application No. |
| --- |
| PCT/JP2006/300813 |

<Subject of search>

    With respect to the terminology "organic compound" recited in claim 1, etc., even after reviewing of the contents of the description, what particular compounds other than compounds of $CH_3(OCH_2CH_2)_6SH$, etc. mentioned as examples in the description are comprehended or not comprehended therein cannot be stated as being clear, thereby causing the scope of the invention of this application to be unclear.

    Further, with respect to the "organic compounds" other than $CH_3(OCH_2CH_2)_6SH$, etc., it can be stated that the disclosure within the meaning of PCT Article 5 is lacking and that the requirement of clarity and support by the disclosure in the description within the meaning of PCT Article 6 are lacking.

    Therefore, as claim 1, etc. and the description fail to satisfy prescribed requirements to such an extent that no meaningful search can be carried out, prior art literature search in this international search report has been carried out on the basis of organic compounds particularly mentioned in the description.

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002128523 A
- WO 1998040049 A
- JP 9110722 A
- WO 2004083124 A
- US 5514394 A
- JP 2619037 B
- JP 5245357 A
- JP 9003093 A
- JP 7165770 A
- JP 2002037883 A
- JP 11106391 A

**Non-patent literature cited in the description**

- *BIO INDUSTRY,* 2004, vol. 21 (8), 48-54
- *Nature Materials,* 2004, vol. 3 (12), 891-895